# EUROPEAN PATENT APPLICATION

(11) **EP 4 759 329 A1**
(43) Date of publication of application: **17.06.2026**
(21) Application number: 24383351.4
(22) Date of filing: 10.12.2024
(51) Int. Cl.: A61K 47/68, A61P 35/00, A61P 35/04, C07K 16/40

(54) **COMBINATION THERAPY FOR THE TREATMENT OF METASTATIC CANCER**

(71) Applicant: Oncomatryx Biopharma, S.L., 48160 Derio (Vizcaya) (ES)
(72) Inventor: GARCÍA RIBAS, Ignacio, Derio (Bilbao), Vizcaya, 48160 (ES); FABRE, Myriam, Derio (Bilbao), Vizcaya, 48160 (ES); SIMÓN BUELA, Laureano, Derio (Bilbao), Vizcaya, 48160 (ES)
(74) Representative: Mewburn Ellis LLP

(57) **Abstract**

The present invention relates to a combination of (i) an anti-Fibroblast Activating Protein α (FAP) antibody-cytolysin conjugate having the formula A-(L-D)ₚ or a pharmaceutically acceptable salt or solvate thereof and (ii) an immune checkpoint inhibitor (ICI) for use in a method of treating metastatic cancer in a mammalian subject, wherein A is an anti-FAP antibody that selectively binds FAP, L is a linker, D is a drug comprising a cytolysin and p is 1 to 10, wherein the method comprises simultaneous, sequential or separate administration of the antibody-cytolysin conjugate or the pharmaceutically acceptable salt or solvate thereof and the ICI to the subject, wherein the metastatic cancer comprises a primary cancer and metastasis.

## Description

### Field of the Invention

The present invention relates to a combination of an anti-Fibroblast Activating Protein α (FAP) antibody-cytolysin conjugate or a pharmaceutically acceptable salt or solvate thereof and an immune checkpoint inhibitor for use in a method of treating metastatic cancer in a mammalian subject.

### Background

Immunotherapy such as immune checkpoint inhibitors (ICls) is a promising therapy increasingly used in the treatment of cancer. Immune checkpoints are receptors expressed on immune cells which are involved with regulation of immune homeostasis. These checkpoints can be especially relevant in cancer, where chronic T cell stimulation leads to dysfunction because of interactions between cells expressing immune checkpoint receptors and ligands. By inhibiting these interactions, ICls can reinvigorate the T cells, restoring T cell functionality and anti-cancer activity.

Although ICls have demonstrated significant efficacy in multiple solid tumour types, the effectiveness of ICls as a single agent or in combination with chemotherapy in certain metastatic cancers has, to date, been limited (O'Reilly et al., 2019; Wainberg et al., 2020; Renouf et al., 2022). For example, ICls have shown limited benefit in the treatment of metastatic pancreatic or colorectal cancer (Ye et al, 2023; Sahin et al., 2022). In particular, metastatic colorectal cancer subjects with microsatellite-stable (MSS) tumours have been found to respond poorly to ICI treatment, even when administered in combination with multitargeted tyrosine kinase inhibitors (TKl). This limited ICI response is especially evident for MSS colorectal cancer patients with liver or peritoneal metastases (TKI) (Dung et al., 2015; Overman et al., 2017; Wang et al, 2021; Fukuoka et al., 2020). Immunotherapy in metastatic pancreatic cancer, either as single agent or in combination with chemotherapy, has also shown very limited benefit outside of the rare mismatch repair deficient tumours (O'Reilly et al., 2019; Renouf et al., 2022).

Another form of immunotherapy which represents great promise in the fight against cancer is antibody-drug conjugates (ADCs). ADCs are made of a human, humanized or chimeric recombinant antibody, covalently linked to a cytotoxic drug. The main goal of such a structure is joining the power of small cytotoxic agents (300 to 1000 Da) and the high specificity of tumour-associated antigen (TAA)- targeted MAbs. One such ADC is OMTX705, which is described in WO 2024/023159. OMTX705 is an anti-Fibroblast Activating Protein α (FAP)-targeted antibody-drug conjugate, wherein the antibody is conjugated to a drug comprising cytolysin.

Despite the success of these immunotherapies in the treatment of some primary cancers, many subjects with metastatic gastrointestinal tumours, especially pancreatic and colorectal metastatic cancer, have not responded to treatment with single agents such as ICls or ADCs such as OMTX705. Therefore, there remains a need for improved treatment of metastatic cancer.

The invention has been devised with the above issues in mind.

### Summary of the Invention

Broadly, the present invention relates to a combination of an anti-FAP antibody-cytolysin conjugate of the type disclosed in WO 2024/023159 (incorporated herein by reference in its entirety) and an immune checkpoint inhibitor (ICI) for use in a method of treating metastatic cancer in a mammalian subject. The present inventors have found that this combination exhibits strong anti-tumour activity in metastatic cancers including non-small cell lung cancer and gastrointestinal cancers such as metastatic colorectal cancer and pancreatic cancer. This anti-tumour activity includes a reduction in carcinoembryonic (CEA) levels, reduction in tumour lesion size and improved progression free and overall survival time. Such activity is surprising, since each therapy exhibits limited or no efficacy as a single agent.

Accordingly, in a first aspect, the present invention provides a combination of (i) an anti-Fibroblast Activating Protein α (FAP) antibody-cytolysin conjugate having the formula A-(L-D)ₚ or a pharmaceutically acceptable salt or solvate thereof, wherein A is an anti-FAP antibody that selectively binds FAP, L is a linker, D is a drug comprising a cytolysin and p is 1 to 10, and (ii) an immune checkpoint inhibitor (ICI) for use in a method of treating metastatic cancer in a mammalian subject, wherein the method comprises simultaneous, sequential or separate administration of the antibody-cytolysin conjugate or the pharmaceutically acceptable salt or solvate thereof and the ICI to the subject, wherein the metastatic cancer comprises a primary cancer and metastasis.

In some embodiments, the primary cancer of the metastatic cancer comprises gastrointestinal cancer or lung cancer. The primary cancer of the metastatic cancer may comprise gastrointestinal cancer.

In some embodiments the ICI comprises an anti-PD-1 molecule, an anti-PD-L1 molecule or an anti-CTLA-4 molecule. In some embodiments the ICI comprises an anti-PD-1 molecule. The anti-PD-1 molecule may comprise pembrolizumab, nivolumab or tislelizumab. Optionally, the anti-PD-1 molecule comprises pembrolizumab.

In some embodiments, A of the antibody-cytolysin conjugate has a heavy chain with amino acid sequence of SEQ ID NO: 1 and a light chain with amino acid sequence of SEQ ID NO: 2.

In some embodiments, the cytolysin of the antibody-cytolysin conjugate comprises formula IV: 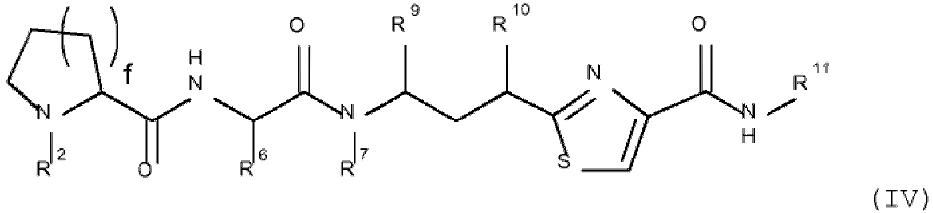 wherein:
R² is H or C₁-C₄ alkyl;
R⁶ is C₁-C₆ alkyl;
R⁷ is C₁-C₆ alkyl, CH₂OR¹⁹ or CH₂OCOR²⁰, wherein R¹⁹ is alkyl, R²⁰ is C₂-C₆-alkenyl, phenyl, or CH₂-phenyl;
R⁹ is C₁-C₆ alkyl;
R¹⁰ is H, OH, O-alkyl or O-acetyl;
f is 1 or 2;
R¹¹ has the following structure: 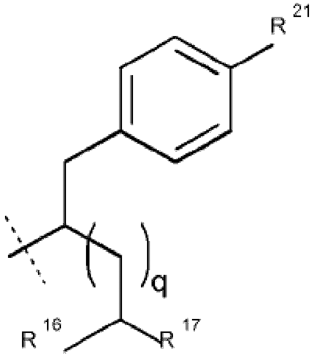
wherein
R²¹ is H, OH, halogen, NH₂, alkyloxy, phenyl, alkyl amino or dialkyl amino;
R¹⁶ is H or a C₁-C₆-alkyl group;
R¹⁷ is directly or indirectly attached to linker L; and
q is 0, 1, 2 or 3;
and wherein the term "optionally substituted" relates to groups, wherein one or several H atoms can be replaced by F, Cl, Br or I or OH, SH, NH₂, or NO₂; the term "optionally substituted" further relates to groups, which can be exclusively or additionally substituted with unsubstituted C₁-C₆ alkyl, C₂C₆ alkenyl, C₂-C₆ alkynyl, C₁-C₆ heteroalkyl, C3-C₁₀ cycloalkyl, C₂-C₉ heterocycloalkyl, C₆-C₁₀ aryl, C₁-C₉ heteroaryl, C₇-C₁₂ aralkyl or C₂-C11 heteroaralkyl groups.

In some embodiments, L of the antibody-cytolysin conjugate comprises a spacer. Optionally, the spacer comprises -(OCH₂CH₂)ₙ-, wherein n is 2 to 5.

In some embodiments, L of the antibody-cytolysin conjugate comprises an attachment group for attachment to A. Optionally, L comprises a protease cleavable portion comprising a valine-citrulline unit.

In some embodiments, the cytolysin of the antibody-cytolysin conjugate has the formula: 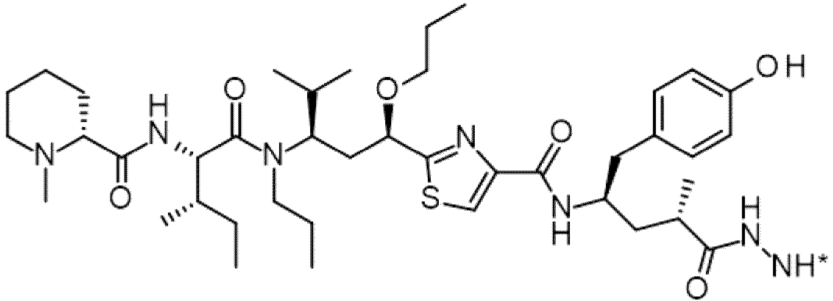 wherein * indicates the site of attachment to L.

In some embodiments -L-D of the antibody-cytolysin conjugate has the structure: 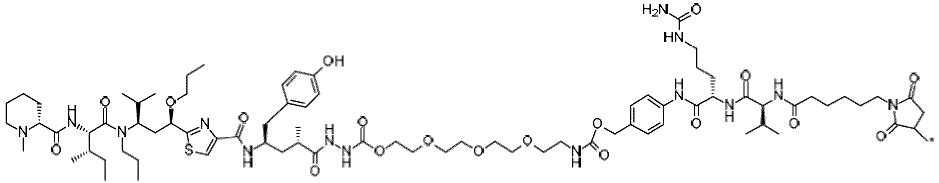 wherein * denotes the point of attachment to A.

In some embodiments, the mammalian subject is a human subject.

In some embodiments the primary cancer comprises pancreatic cancer, colorectal cancer, oesophageal cancer, gastric (stomach) cancer, liver cancer or lung cancer.

The primary cancer may comprise pancreatic cancer, colorectal cancer, oesophageal cancer or lung cancer.

The primary cancer may comprise pancreatic, colorectal or oesophageal cancer. Alternatively, the primary cancer may comprise pancreatic, colorectal or lung cancer.

The pancreatic cancer may comprise pancreatic ductal adenocarcinoma (PDAC).

In some embodiments, the colorectal cancer comprises microsatellite-stable (MSS) colorectal cancer.

In some embodiments the primary cancer comprises MSS colorectal cancer and the metastasis comprises liver, lung and/or peritoneal metastasis.

The lung cancer may comprise non-small cell lung cancer (NSCLC).

In some embodiments, the primary cancer is selected from pancreatic ductal adenocarcinoma (PDAC), colorectal cancer, oesophageal cancer and non-small cell lung cancer (NSCLC).

In some embodiments, the subject was previously administered the antibody-cytolysin conjugate and was not previously administered the ICI. In other embodiments, the subject was previously administered the ICI and was not previously administered the antibody-cytolysin conjugate.

In some embodiments the method comprises sequential administration of the antibody-cytolysin conjugate and the ICI to the subject.

In some embodiments, the method comprises intravenous administration of the antibody-cytolysin conjugate and the ICI to the subject.

The method may comprise administration of the antibody-cytolysin conjugate at least once in a 21-day cycle. In some embodiments the method comprises administration of the antibody-cytolysin conjugate at least twice in a 21-day cycle. In some embodiments the method comprises administration of the antibody-cytolysin conjugate on day 1 and day 8 of a 21-day cycle. In some embodiments the method comprises administration of the ICI at least once in a 21-day cycle. In some embodiments the method comprises administration of the ICI on day 1 of a 21-day cycle. In some embodiments the 21-day cycle is repeated at least once.

In some embodiments administration of the antibody-cytolysin conjugate and the ICI results in a synergistic effect.

Administration of the antibody-cytolysin conjugate and the ICI may result in a reduction of tumour lesion size in the subject, preferably metastatic tumour lesion size. In some embodiments, administration of the antibody-cytolysin conjugate and the ICI results in a reduction of primary tumour and metastatic tumour lesion size.

In some embodiments, administration of the antibody-cytolysin conjugate and the ICI increases progression free survival and/or overall survival time of the subject relative to a control subject who has been treated with the antibody-cytolysin conjugate or the ICI as monotherapies but not in combination.

In some embodiments, the method comprises administration of at least 2mg/kg of the antibody-cytolysin conjugate to the subject. Preferably, the method comprises administration of at least 4mg/kg of the antibody-cytolysin conjugate to the subject. In some embodiments, the method comprises administration of at least 7mg/kg of the antibody-cytolysin conjugate to the subject.

In some embodiments the method comprises administration of at least 100mg, at least 150mg, at least 200mg, at least 250mg, at least 300mg, at least 350mg or at least 400mg of the ICI to the subject.

The method may comprise administration of 200mg of ICI to the subject.

In some embodiments the primary cancer and/or metastasis comprises a level of FAP expression detectable by immunohistochemistry.

In a further aspect, the invention provides a method of treating metastatic cancer in a mammalian subject, the method comprising administering (i) an anti-Fibroblast Activating Protein α (FAP) antibody-cytolysin conjugate having the formula A-(L-D)ₚ or a pharmaceutically acceptable salt or solvate thereof, wherein A is an anti-FAP antibody that selectively binds FAP, L is a linker, D is a drug comprising a cytolysin and p is 1 to 10, to the subject simultaneously, sequentially or separately with (ii) an immune checkpoint inhibitor (ICI), wherein the metastatic cancer comprises a primary cancer and metastasis.

The primary cancer of the metastatic cancer may comprise gastrointestinal or lung cancer.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1****.** An illustrative depiction of the conjugate OMTX705, as described in Fabre et al. (2020). As shown, OMTX705 comprises a humanised anti-FAP mAb (OMTX005) conjugated to TAM558, which is formed from the conjugation of the cytolysin TAM470 to a protease-cleavable vcPABA-(EG)ₛ optimised linker.
**Figure 2****.** Schematic to show dose-escalation protocols used when comparing OMTX705 monotherapy to OMTX705 + ICI combination therapy.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### Antibody-cytolysin conjugate

The anti-FAP antibody-cytolysin conjugate of the invention has the formula I:

A-(L-D)ₚ

wherein A is an anti-FAP antibody that selectively binds FAP, L is a linker, D is a drug comprising a cytolysin and p is 1 to 10.

As used herein "Fibroblast activation protein", "fibroblast activating protein", "FAP" and "FAPα" are used interchangeably. The FAP may be an FAP of any mammalian species. In some cases FAP is human FAP (also known as Seprase, 170 kDa melanoma membrane-bound gelatinase, fibroblast activation protein alpha or integral membrane serine protease), the amino acid sequence of which is disclosed at UniProt accession No. Q12884 (Version 140, dated 11 December 2013) (SEQ ID NO: 13). In some cases, a molecule that binds FAP (e.g. an antibody molecule or a conjugate thereof) may bind to a region of the extracellular domain of FAP. The extracellular domain of human FAP comprises residues 26-760 of the full-length human FAP protein. In some cases FAP is murine FAP (also known as fibroblast activation protein alpha or integral membrane serine protease), the amino acid sequence of which is disclosed at UniProt accession No. P97321 (Version 117, dated 11 December 2013) (SEQ ID NO: 14). The extracellular domain of murine FAP comprises residues 26-761 of the full-length murine FAP protein.

In the context of the present invention, the terms selectively binds and selective binding refer to binding of an antibody, or binding fragment thereof, to a predetermined molecule (e.g. an antigen) in a specific manner. For example, the antibody, or binding fragment thereof, may bind to FAP, e.g. an extracellular portion thereof, with an affinity of at least about 1x10⁷M⁻¹, and may bind to the predetermined molecule with an affinity that is at least two-fold greater (e.g. five-fold or ten-fold greater) than its affinity for binding to a molecule other than the predetermined molecule.

As used herein the term "antibody" or "antibody molecule" includes any immunoglobulin whether natural or partly or wholly synthetically produced. The term "antibody" or "antibody molecule" includes monoclonal antibodies (mAb) and polyclonal antibodies (including polyclonal antisera). Antibodies may be intact or fragments derived from full antibodies (see below). Antibodies may be human antibodies, humanised antibodies or antibodies of non-human origin. "Monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule. The term "antiserum" or "antisera" refers to blood serum containing antibodies obtained from immunized animals.

It has been shown that fragments of a whole antibody can perform the function of binding antigens. Thus reference to antibody herein covers a full antibody and also covers any polypeptide or protein comprising an antibody binding fragment. Examples of binding fragments are (i) the Fab fragment consisting of V_{L}, V_{H}, C_{L} and C_{H}1 domains; (ii) the Fd fragment consisting of the V_{H} and C_{H}1 domains; (iii) the Fv fragment consisting of the V_{L} and V_{H} domains of a single antibody; (iv) the dAb fragment which consists of a V_{H} domain; (v) isolated CDR regions; (vi) F(ab')₂ fragments, a bivalent fragment comprising two linked Fab fragments (vii) single chain Fv molecules (scFv), wherein a V_{H} domain and a V_{L} domain are linked by a peptide linker which allows the two domains to associate to form an antigen binding site; (viii) bispecific single chain Fv dimers (WO 93/11161) and (ix) "diabodies", multivalent or multispecific fragments constructed by gene fusion (WO94/13804; 58). Fv, scFv or diabody molecules may be stabilised by the incorporation of disulphide bridges linking the VH and VL domains. Minibodies comprising a scFv joined to a CH3 domain may also be made.

In relation to an antibody molecule, the term "selectively binds" may be used herein to refer to the situation in which one member of a specific binding pair will not show any significant binding to molecules other than its specific binding partner(s). The term is also applicable where e.g. an antigen-binding site is specific for a particular epitope that is carried by a number of antigens, in which case the specific binding member carrying the antigen-binding site will be able to bind to the various antigens carrying the epitope.

In some embodiments, the anti-FAP antibody is a monoclonal antibody or binding fragment thereof that selectively binds to an extracellular region of human FAP and/or murine FAP. In some embodiments, the anti-FAP antibody cross-reacts to both human and murine FAP.

In some embodiments the antibody may comprise a humanised antibody.

In some embodiments the antibody may be a fully human antibody.

In some embodiments, the anti-FAP antibody comprises heavy chain complementarity determining regions 1-3 (CDRH1-3) and light chain complementarity determining regions 1-3 (CDRL1-3) having the following amino acid sequences:
(i) CDRH1: SEQ ID NO: 7;
(ii) CDRH2: SEQ ID NO: 8;
(iii) CDRH3: SEQ ID NO: 9;
(iv) CDRL1: SEQ ID NO: 10;
(v) CDRL2: SEQ ID NO: 11; and
(vi) CDRL3: SEQ ID NO: 12.

In some embodiments, the CDRH1 region comprises a variant of SEQ ID NO: 7 having up to 1 or 2 amino acid substitutions compared with the sequence of SEQ ID NO: 7. In some embodiments, the CDRH2 region comprises a variant of SEQ ID NO: 8 having up to 1 or 2 amino acid substitutions compared with the sequence of SEQ ID NO: 8. In some embodiments, the CDRH3 region comprises a variant of SEQ ID NO: 9 having up to 1 or 2 amino acid substitutions compared with the sequence of SEQ ID NO: 9. In some embodiments, the CDRL1 region comprises a variant of SEQ ID NO: 10 having up to 1 or 2 amino acid substitutions compared with the sequence of SEQ ID NO: 10. In some embodiments, the CDRL2 region comprises a variant of SEQ ID NO: 11 having up to 1 or 2 amino acid substitutions compared with the sequence of SEQ ID NO: 11. In some embodiments, the CDRL3 region comprises a variant of SEQ ID NO: 12 having up to 1 or 2 amino acid substitutions compared with the sequence of SEQ ID NO: 12.

In some embodiments, CDRH1-3 comprise the amino acid sequences of SEQ ID NOS: 7-9, respectively and CDRL1-3 comprise the amino acid sequences of SEQ ID NOS: 10-12, respectively.

In some embodiments, the anti-FAP antibody comprises a heavy chain variable region (VH) comprising the amino acid sequence of SEQ ID NO: 5 and a light chain variable region (VL) comprising the amino acid sequence of SEQ ID NO: 6. In some embodiments, the heavy chain variable region (VH) has at least 90%, 95% or 99% sequence identity with the full-length sequence of SEQ ID NO: 5. In some embodiments, the light chain variable region (VL) has at least 90%, 95% or 99% sequence identity with the full-length sequence of SEQ ID NO: 6.

In some embodiments, the anti-FAP antibody comprises a heavy chain comprising the amino acid sequence of SEQ ID NO: 1 and a light chain comprising the amino acid sequence of SEQ ID NO: 2. In some embodiments, the heavy chain has at least 90%, 95% or 99% sequence identity with the full-length sequence of SEQ ID NO: 1. In some embodiments, the light chain has at least 90%, 95% or 99% sequence identity with the full-length sequence of SEQ ID NO: 2.

In some embodiments, the anti-FAP antibody comprises a heavy chain comprising the amino acid sequence of SEQ ID NO: 3 and a light chain comprising the amino acid sequence of SEQ ID NO: 4. In some embodiments, the heavy chain has at least 90%, 95% or 99% sequence identity with the full-length sequence of SEQ ID NO: 3. In some embodiments, the light chain has at least 90%, 95% or 99% sequence identity with the full-length sequence of SEQ ID NO: 4.

In some embodiments, A of the antibody-cytolysin conjugate has a heavy chain with amino acid sequence of SEQ ID NO: 1 and a light chain with amino acid sequence of SEQ ID NO: 2.

The cytolysin of the antibody-cytolysin conjugate may be a compound disclosed in WO 2008/138561 A1, the entire contents of which is expressly incorporated herein by reference (compounds disclosed therein are also referred to as Tubulysine derivatives). The cytolysin may be synthesised as described in WO 2008/138561. In some embodiments, the cytolysin is as defined in Formula I or Formula IV of WO 2008/138561 A1. In some embodiments, the cytolysin is of formula IV: 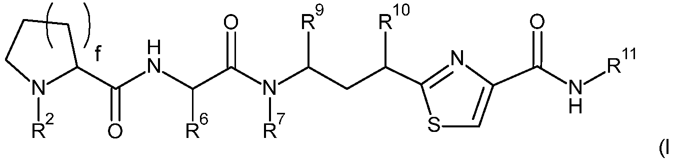 wherein:
R² is H or is C₁-C₄ alkyl;
R⁶ is C₁-C₆ alkyl;
R⁷ is C₁-C₆ alkyl, CH₂OR¹⁹ or CH₂OCOR²⁰, wherein R¹⁹ is alkyl, R²⁰ is C₂-C₆-alkenyl, phenyl, or CH₂-phenyl;
R⁹ is C₁-C₆ alkyl;
R¹⁰ is H, OH, O-alkyl or O-acetyl;
f is 1 or 2;
R¹¹ has the following structure: 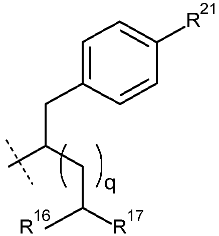
wherein
R²¹ is H, OH, halogen, NH₂, alkyloxy, phenyl, alkyl amino or dialkyl amino;
R¹⁶ is H or a C₁-C₆-alkyl group;
R¹⁷ is directly or indirectly attached to linker L; and
q is 0, 1, 2 or 3;
and wherein the term "optionally substituted" relates to groups, wherein one or several H atoms can be replaced by F, Cl, Br or I or OH, SH, NH₂, or NO₂; the term "optionally substituted" further relates to groups, which can be exclusively or additionally substituted with unsubstituted C₁-C₆ alkyl, C₂C₆ alkenyl, C₂-C₆ alkynyl, C₁-C₆ heteroalkyl, C₃-C₁₀ cycloalkyl, C₂-C₉ heterocycloalkyl, C₆-C₁₀ aryl, C₁-C₉ heteroaryl, C₇-C₁₂ aralkyl or C₂-C₁₁ heteroaralkyl groups.

In some embodiments R² is a bond to linker L.

In some embodiments R¹⁷ is C(O)X, CONHNHX, OX, NHX or SX, wherein X is a bond to linker L.

In some embodiments, linker L comprises a spacer. In some embodiments the spacer has a chain length of 2 to 30 atoms. In some embodiments the spacer comprises or consists of an alkylene (i.e. divalent alkyl) or heteroalkylene (i.e. divalent heteroalkyl) group. In some embodiments the spacer comprises or consists of an alkylene or oxyalkylene group.

In some embodiments, the spacer comprises -(OCH₂CH₂)ₙ-, wherein n is 2 to 5. In some embodiments the spacer comprises or consists of a group -(CH₂)ₙ- or -(OCH₂CH₂)ₙ-, wherein n ≥ 1. In some embodiments the spacer comprises or consists of a group -(OCH₂CH₂)ₙ-, wherein n ≥ 1. In particular, n may be 1 to 15, 1 to 10, 1 to 6, or 2 to 5. For example, n may be 3 or 4. In some embodiments the spacer comprises between one and six ethylene glycol units, e.g. a triethylene glycol. In some embodiments the spacer may be directly attached to group R¹⁷, or may be attached to group R¹⁷ via a bridging group. In some embodiments the spacer is attached to group R¹⁷ via a -C(O)X bridging group, wherein X is a bond to R¹⁷. In some embodiments R¹⁷ is CONHNHX and the spacer is attached to group R¹⁷ via a -C(O)X bridging group, wherein X represents the bond between the spacer and R¹⁷. In some embodiments R¹⁷ is CONHNHX and the spacer is a -(OCH₂CH₂)ₙ- attached to R¹⁷ via a -C(O)X bridging group, wherein n = 2, 3 or 4.

In some embodiments, L comprises an attachment group for attachment to A.

In some embodiments, L comprises a protease cleavable portion comprising a valine-citrulline unit. For example, L may comprise maleimidocaproyl-valine-citrulline-p-aminobenzylcarbamate.

In some embodiments the cytolysin has the following structure: 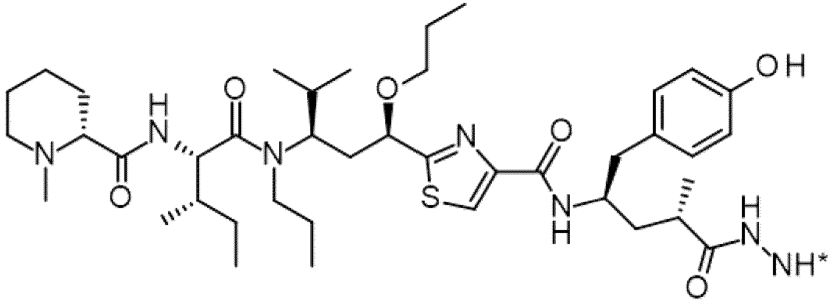 wherein * indicates the site of attachment to L.

In some embodiments D comprises a cytolysin having the following structure:

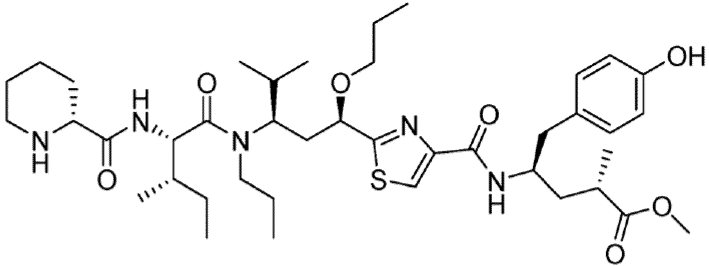

In some embodiments the double bond of the maleimide is reacted with a thiol group of a cysteine residue of the antibody A to form a sulphur-carbon bond in order to effect linkage of the linker L to the antibody A.

In some embodiments -L-D has the structure:

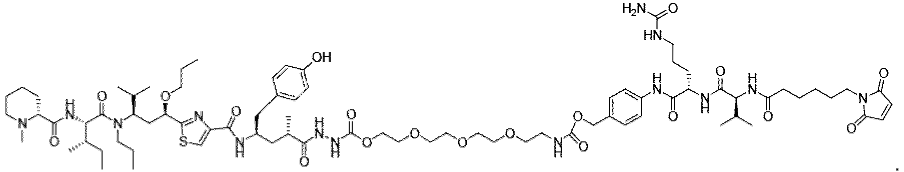

In other embodiments -L-D has a structure selected from the group consisting of: 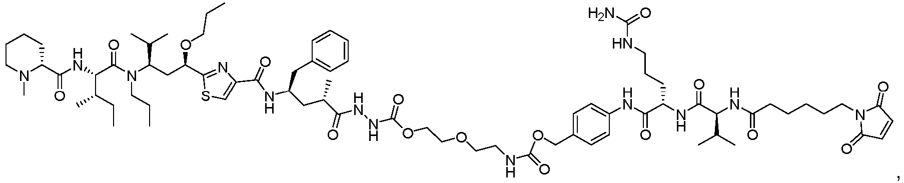 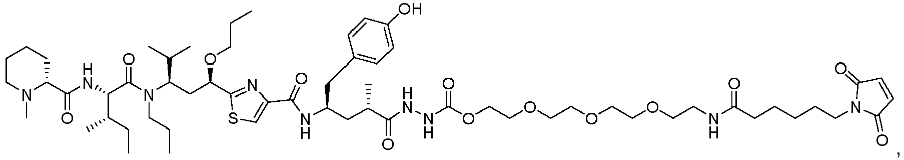 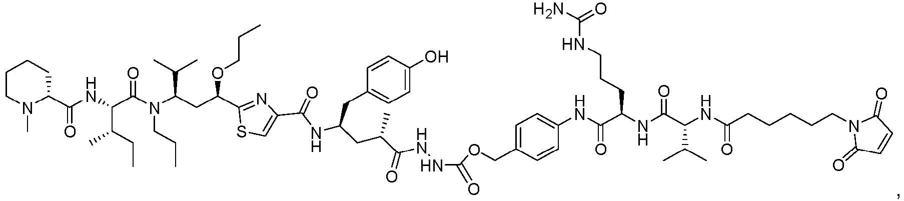 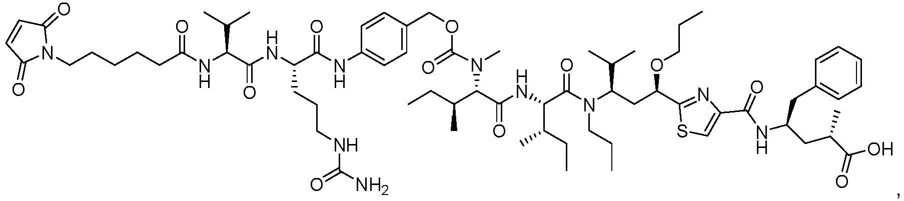 and

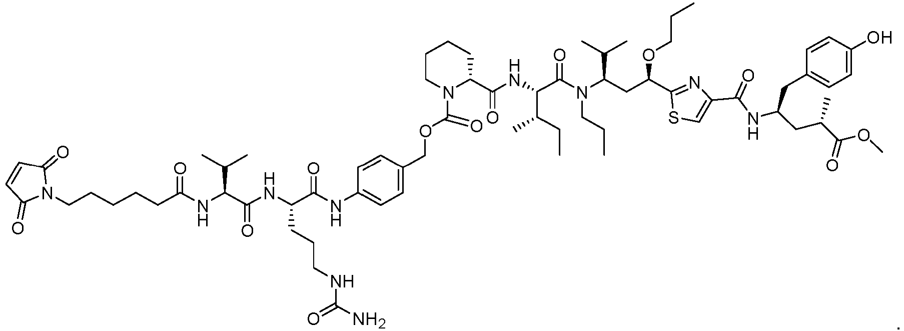

In certain embodiments -L-D has the following structure:

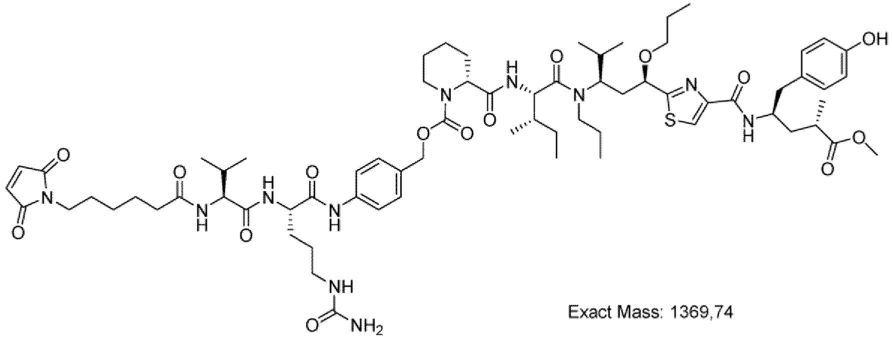

In certain embodiments -L-D has the following structure: 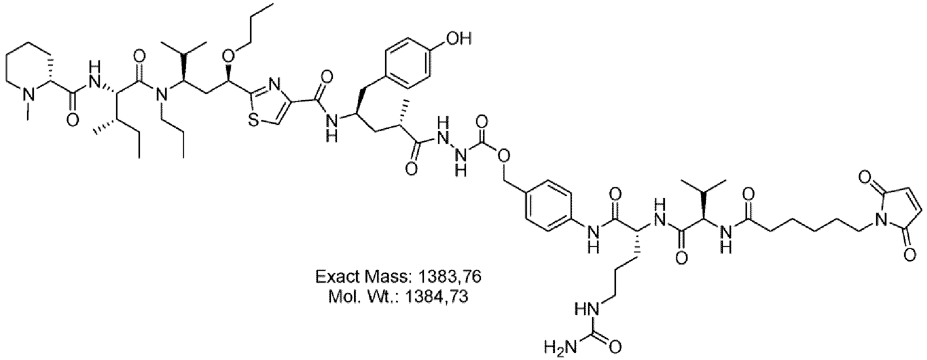 p may, in some embodiments, lie in the range 1 to 5, e.g. 1 to 4, or 1 to 3. In particular embodiments p is 1 or 2. p may be 3 or 4.

In some embodiments, A has a heavy chain with amino acid sequence of SEQ ID NO: 3 and a light chain with amino acid sequence of SEQ ID NO: 4; and L-D has the structure: 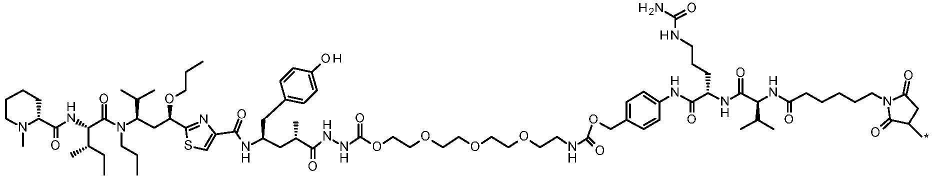 wherein * denotes the point of attachment to A.

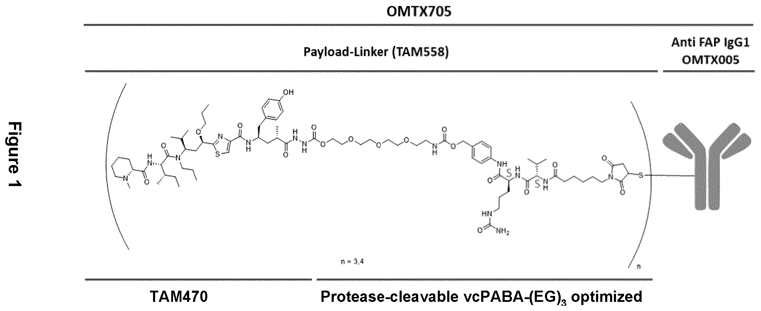

In some embodiments, the anti-FAP antibody-cytolysin conjugate is the antibody-cytolysin conjugate described herein as OMTX705. OMTX705 comprises a humanised anti-FAP mAb (OMTX005) conjugated to the cytolysin TAM470 via a protease-cleavable vcPABA-(EG)₃ optimised linker. OMTX705 is described in Fabre et al. (2020), and Figure 1 is an illustrative depiction of OMTX705.

### Immune Checkpoint Inhibitor (ICI)

Various ICls are known in the art and are suitable for use in the present invention. For example, the ICI may comprise an anti-programmed cell death protein (PD-1) antibody, an anti-programmed death-ligand 1 (PD-L1) antibody or an anti-cytotoxic T-lymphocyte associated protein 4 (CTLA-4) antibody. In some embodiments the ICI comprises an anti-PD-1 antibody or an anti-PD-L1 antibody.

In some embodiments, the anti-PD-L1 antibody comprises durvalumab. In some embodiments, the anti CTLA-4 antibody comprises tremelimumab.

In some embodiments, the ICI comprises an anti-PD-1 antibody. For example, the ICI may comprise nivolumab (MDX1106) or pembrolizumab (MK-3475). In some embodiments the anti-PD-1 antibody comprises tislelizumab. In some embodiments, the anti-PD-1 antibody comprises pembrolizumab. Preferably, the ICI comprises pembrolizumab or tislelizumab.

### Subject

The subject is a mammal. Preferably, the subject is a human, but may be any other primate (great ape, old world monkey or new world monkey), or a domestic, laboratory or livestock animal, such as a mouse, rat, guinea pig, lagomorph (e.g. rabbit), cat, dog, pig, cow, horse, sheep or goat.

The subject may previously have undergone anti-cancer therapy and/or surgery, but may not previously have been administered the specific combination of the present invention. Indeed, the present inventors envisage that the present invention may have particular utility for subjects who are non-responders to other previous treatments and/or surgeries. In particular, the present inventors envisage that the present invention may have particular utility for subjects who are non-responders to previous ICI or antibody-cytolysin conjugate monotherapies (i.e. where only one of the combination therapy has previously been administered to the subject and the cancer is non-responsive to this monotherapy).

In the context of the present invention, a "non-responder" will be understood to refer to a subject whose cancer has not responded to a particular treatment and/or surgery. Typically, the cancer of a "non-responder" will continue to progress despite the treatment and/or surgery. Therefore, a "non-responder" will typically be understood to refer to a subject who has progressive cancer disease despite the treatment and/or surgery.

Thus, in some embodiments, the subject was previously administered the antibody-cytolysin conjugate and was not previously administered the ICI. In some embodiments, the subject was previously administered the antibody-cytolysin conjugate only, i.e. without any other anti-cancer treatment. In other words, the subject may be a non-responder to antibody-cytolysin conjugate monotherapy. In other embodiments, the subject was previously administered the antibody-cytolysin conjugate and at least one other anti-cancer therapy, such as radiotherapy and/or chemotherapy, but was not previously administered the ICI.

By "previously administered", this will be understood to refer to administration prior to the method of the present invention.

In other embodiments, the subject was previously administered the ICI and was not previously administered the antibody-cytolysin conjugate. In some embodiments, the subject was previously administered the ICI only, i.e. without any other anti-cancer treatment. In other words, the subject may be a non-responder to ICI, for example anti-PD-1 molecule monotherapy. The subject may be a non-responder to pembrolizumab, nivolumab or tislelizumab monotherapy. In other embodiments, the subject was previously administered the ICI and at least one other anti-cancer therapy, such as radiotherapy and/or chemotherapy, but was not previously administered the antibody-cytolysin conjugate.

### Cancer

Preferably, the cancer is a carcinoma. As the skilled person will appreciate "carcinoma" refers to cancer of the epithelial tissue.

In the context of the present invention, the term primary cancer refers to the anatomical site where the cancer first starts growing in the subject. Thus, reference to, for example, a primary cancer comprising pancreatic cancer means that the cancer first started growing in the subject in the pancreas. In such embodiments, the primary site of the cancer is the pancreas.

As used herein, the term "metastatic cancer" refers to a primary cancer where the cancer has spread from the primary cancer to another anatomical site in the subject. Thus, in the context of the present invention, metastatic cancer comprises the primary cancer and cancer in an anatomical site of the subject different to the site of the primary cancer. These cancers in sites different to the site of the primary cancer may otherwise be referred to as metastases or metastasis. For the avoidance of doubt, it will be appreciated that reference to "metastases" refers to a plurality of metastasis, i.e. a plurality of secondary tumours which are located in one or more anatomical sites of the subject different to the site of the primary cancer.

Where a cancer is referred to as, for example, "metastatic pancreatic cancer", this will be understood to refer to a cancer where the primary cancer is pancreatic cancer, but this has spread to anatomical sites other than the pancreas in the subject. Likewise, where a cancer is referred to as, for example, "metastatic colorectal cancer", this will be understood to refer to a primary cancer comprising colorectal cancer, which has spread to anatomical sites different to the colorectum in the subject. In such examples, the metastatic cancer comprises both the primary cancer and metastases in anatomical locations different to that of the primary cancer.

Preferably, the primary cancer comprises gastrointestinal or lung cancer. As the skilled person will appreciate, gastrointestinal cancer is cancer of the digestive tract and/or other abdominal organs. Gastrointestinal cancer may comprise pancreatic cancer, colorectal cancer, oesophageal cancer, gastric (stomach) cancer, liver cancer, anal cancer, gastrointestinal stroma cancer, neuroendocrine cancer or cancer of the small intestine.

In some embodiments, the primary cancer comprises lung cancer, pancreatic cancer, colorectal cancer, gastric cancer or oesophageal cancer.

In some embodiments, the primary cancer comprises pancreatic cancer, colorectal cancer, gastric cancer or oesophageal cancer.

The lung cancer may comprise non-small cell lung cancer (NSCLC).

In some embodiments, the primary cancer comprises pancreatic cancer or colorectal cancer. In some embodiments, the primary cancer comprises pancreatic cancer. In some embodiments, the primary cancer comprises colorectal cancer. The pancreatic cancer may comprise pancreatic ductal adenocarcinoma (PDAC), pancreatic squamous cell carcinoma or pancreatic adenosquamous carcinoma. In some embodiments the pancreatic cancer comprises PDAC.

The colorectal cancer may comprise microsatellite-stable (MSS) colorectal cancer. Micro-satellite stable colorectal cancer will be understood to refer to colorectal cancer wherein the microsatellite DNA segments in the colorectal cancer are not mutated. Thus, in some embodiments, the colorectal cancer comprises microsatellite-stable DNA segments detectable by immunohistochemistry (IHC), PCR or next-generation sequencing. The present inventors have found that the combination of an ICI and an antibody-cytolysin conjugate has especially effective anti-tumour activity against metastatic pancreatic cancer and metastatic MSS colorectal cancer. This is surprising given that each therapy individually does not show efficacy against these tumour types.

In some embodiments, the primary cancer is selected from PDAC, MSS colorectal cancer, NSCLC and oesophageal cancer.

The metastasis or metastases of the metastatic cancer may comprise any metastasis or metastases in an anatomical site different to that of the primary cancer. For example, the metastasis may comprise lung, liver, lymph node, peritoneal, bone and/or bile duct metastasis/metastases.

In some embodiments the metastasis comprises liver, peritoneal, lymph node and/or lung metastasis. The metastasis may comprise liver metastasis. The metastasis may comprise peritoneal metastasis. In some embodiments, the metastasis comprises liver, lung and/or peritoneal metastasis. In some embodiments, the metastasis comprises liver or peritoneal metastasis. In some embodiments the metastasis comprises liver or lung metastasis. In some embodiments, the metastasis comprises liver and peritoneal metastases.

In particular embodiments, the primary cancer comprises MSS colorectal cancer and the metastasis comprises liver, lung and/or peritoneal metastasis. Advantageously, the combination of the ICI and the antibody cytolysin conjugate demonstrates surprisingly effective anti-tumour activity in MSS colorectal cancer with liver, lung and/or peritoneal metastasis.

It will be appreciated that some subjects may have been diagnosed with metastatic cancer, due to the presence of multiple tumours in a plurality of different anatomical locations in the body, but that it is not possible to determine the location of the primary cancer and the location of the metastases. In such instances, it is only possible to determine the location of tumours, and not what was the primary source of the tumours. In such embodiments, the primary cancer and metastasis may reside in at least two of the following anatomical locations: lung, liver, lymph node, peritoneum, bone, bile duct, pancreas, colon, rectum, oesophagus, stomach, and small intestine. For example, the primary cancer and metastasis may reside in at least two of the following anatomical locations: lung, liver, lymph node, peritoneum, pancreas, colon and rectum.

The primary cancer and/or metastasis may comprise a level of FAP expression detectable by immunohistochemistry. The level of FAP expression may be detectable from a biological sample taken from the primary cancer and/or metastasis. The biological sample may otherwise be referred to as a biopsy.

In some embodiments, administration of the antibody-cytolysin conjugate and the ICI results in a synergistic effect. The inventors have surprisingly found that while each therapy individually may not have an anti-tumour effect, the combination of the two can provide effective anti-tumour activity for metastatic cancer.

In the context of the present invention, the term "anti-tumour activity" will be understood to refer to activity which reduces symptoms of the cancer, reduces number and/or size of tumours, and/or increases progression free survival and/or overall survival time of the subject. Typically, the subject will be considered a responder to the combination, such that their condition improves, or at least remains stable. The condition remaining stable may otherwise be referred to as stable disease.

A reduced number and/or size of tumour may comprise a reduction of at least about 5%, at least about 10%, at least about 15%, at least about 20%, at least about 25%, at least about 30%, at least about 35%, at least about 40%, at least about 45% or at least about 50% relative to the number and/or size of the tumour prior to administration of the combination. The reduction in number and/or size of tumour may be relative to a baseline value for the number and/or size of the tumour, as discussed further below. In some embodiments, administration of the antibody-cytolysin conjugate and the ICI results in an at least 30% reduction of tumour size and/or number. A reduced number and/or size of tumour may comprise a reduction of at least about 5% to at least about 100%. A 100% reduction will be understood to mean that there are 0 detectable tumours and/or no detectable sites of tumours remaining. For example, where a subject may have five identified tumours prior to administration of the combination, a 100% reduction will be understood to mean that the subject, as a result of the administration, has 0 identified tumours. The anti-tumour activity will be understood to apply to the metastasis and/or the primary cancer.

The criteria used to determine objective tumour response for target lesions/ tumours is well known from the RECIST1.1 and iRECIST studies (RECIST: Eisenhauer EA, Therasse P, Bogaerts J, Schwartz LH, Sargent D, Ford R, Dancey J, Arbuck S, Gwyther S, Mooney M, Rubinstein L, Shankar L, Dodd L, Kaplan R, Lacombe D, Verweij J. New response evaluation criteria in solid tumours: revised RECIST guideline (version 1.1). Eur J Cancer. 2009 Jan;45(2):228-47. doi: 10.1016/j.ejca.2008.10.026. PMID: 19097774 and iRECIST: Seymour L, Bogaerts J, Perrone A, Ford R, Schwartz LH, Mandrekar S, Lin NU, Litière S, Dancey J, Chen A, Hodi FS, Therasse P, Hoekstra OS, Shankar LK, Wolchok JD, Ballinger M, Caramella C, de Vries EGE; RECIST working group. iRECIST: guidelines for response criteria for use in trials testing immunotherapeutics. Lancet Oncol. 2017 Mar;18(3):e143-e152. doi: 10.1016/S1470-2045(17)30074-8. Epub 2017 Mar 2. Erratum in: Lancet Oncol. 2019 May;20(5):e242. doi: 10.1016/S1470-2045(19)30240-2. PMID: 28271869; PMCID: PMC5648544). The entirety of each of these references is incorporated herein. A target lesion may otherwise be referred to as a tumour or tumour lesion.

In the context of the present invention, a responder may comprise a partial responder. A partial responder is a subject who has a "partial response" to the combination. As defined in RECIST1.1 and iRECIST, a partial response will be understood to refer to at least a 30% decrease in the sum of diameters and/or number of target lesions, taking as reference the baseline sum diameters or number of lesions.

Progressive cancer disease, which may otherwise be referred to as progressive disease, will be understood to mean that the subject has at least a 20% increase in the sum of diameters and/or number of tumour lesions compared to a baseline sum diameter or number of tumour lesions.

The baseline sum diameter or number of tumour lesions may comprise the sum diameter or number of tumour lesions measured on day 1 of the cycle, or prior to the combination treatment commencing. Alternatively, the baseline sum diameter or number of tumour lesions may comprise a previous lowest value of sum diameter or number of tumour lesions measured. In such embodiments, progressive disease occurs when the sum of diameters or number of tumour lesions is 20% higher than the previous lowest value. For example, a subject may have a sum of diameters prior to treatment of 110, with the sum of diameters reducing after treatment to 100. In embodiments where the baseline comprises the previous lowest value, the baseline for this subject will be 100. Thus, progressive disease will be reached if and when the sum of diameters reaches 120 (20% increase upon 100). The appearance of one or more new lesions may be defined as progressive disease.

As defined in RECIST 1.1 and iRECIST, stable disease will be understood to refer to neither sufficient shrinkage to qualify for partial response nor sufficient increase to qualify for progressive disease. Thus, stable disease will be understood to refer to metastatic cancer wherein the tumours of the primary and/or metastatic cancer have increased by less than 20% in size versus the smallest size of the tumour prior to administration of the combination or wherein the number of tumour lesions has not increased compared to the number prior to administration of the combination. Stable disease may therefore be defined as metastatic cancer wherein the tumours of the primary and/or metastases have increased by less than 20% in size compared to a baseline tumour size. Stable disease may be defined as metastatic cancer wherein the tumours do not have unequivocal progression as assessed by the treating physician or by an external expert radiologists. Therefore, stable disease is not progressive disease and allows therapy to continue.

In some embodiments, administration of the antibody-cytolysin conjugate and the ICI results in a reduction of tumour burden in the subject. As used herein, the term tumour burden defines the total amount of cancer in the subject.

The size or volume of tumours in the subject may be calculated using RECIST 1.1 and/or iRECIST criteria. For example, the size or volume of tumours in the subject may be calculated as the total sum of long axis diameters of the tumours in the primary tumour and the metastatic tumour(s). For lymph nodes >15 mm, the short axis measurement may be used instead. The total sum of diameters of the tumours in the primary tumour and the metastatic tumour(s) may be measured prior to administration of the combination treatment to provide a baseline value. The total sum of diameters of the tumours in the primary tumour and the metastatic tumour(s) may be measured after administration of the combination treatment to provide a post-treatment value. The difference between the baseline value and the post-treatment value may be calculated as a percentage or ratio, which may then be used to determine if the tumour size is reduced, the same or increased following combination treatment. Typically, if the tumour size has not increased more than 20% compared to the tumour size prior to administration of the combination treatment and the number of tumour lesions has not increased, this will be understood to mean that the cancer is stable disease or has at least partially responded to treatment.

In some embodiments, a reduction of tumour size comprises a reduction in the size or volume of tumours. In some embodiments, a reduction of tumour size comprises a reduction in metastatic tumour lesion size. In some embodiments a reduction of tumour size comprises a reduction in primary tumour lesion size. Preferably, a reduction of tumour size comprises a reduction in primary and metastatic tumour lesion size.

In some embodiments, administration of the antibody-cytolysin conjugate and the ICI reduces carcinoembryonic (CEA) levels in the subject, relative to the CEA level in the subject prior to administration of the combination. The CEA level may be determined from a blood sample obtained from the subject prior to and after combination treatment. The CEA level may be reduced by at least about 10%, at least about 20%, at least about 30%, at least about 40%, or at least about 50%. In some embodiments, administration of the antibody-cytolysin conjugate and the ICI reduces the CEA level in the subject relative to the CEA level in the subject prior to administration of the combination of from about 10% to about 100%.

In some embodiments, administration of the antibody-cytolysin conjugate and the ICI reduces CA19.9 levels in the subject, relative to the CA19.9 level in the subject prior to administration of the combination. The CA19.9 level may be determined from a blood sample obtained from the subject prior to and after combination treatment. The CA19.9 level may be reduced by at least about 10%, at least about 20%, at least about 30%, at least about 40%, or at least about 50%. In some embodiments, administration of the antibody-cytolysin conjugate and the ICI reduces the CA19.9 level in the subject relative to the CA19.9 level in the subject prior to administration of the combination of from about 10% to about 100%.

In some embodiments, administration of the antibody-cytolysin conjugate and the ICI increases progression free survival and/or overall survival time of the subject relative to a control subject who has been treated with the antibody-cytolysin conjugate or with the ICI, as a monotherapy, but not in combination.

"Treated as a monotherapy" will be understood to mean that the control subject has, for example, previously been treated with the antibody-cytolysin conjugate but not the ICI, or with the ICI but not the antibody-cytolysin conjugate.

In some embodiments, administration of the antibody-cytolysin conjugate and the ICI increases progression free survival and/or overall survival time of the subject. In some embodiments, progression free survival and/or overall survival time is increased by at least about 50%, at least about 100% or at least about 200% relative to a control subject who has been treated with the antibody-cytolysin conjugate and not treated with the ICI.

### Administration

In some embodiments, the method comprises simultaneous, sequential or separate administration of the antibody-cytolysin conjugate and the ICI. Preferably, the method comprises sequential administration of the antibody-cytolysin conjugate and the ICI to the subject. Sequential administration, as used herein, will be understood to refer to an administration regime where one agent is administered directly after administration of another agent. Thus, in some embodiments, the method comprises sequential administration of first the antibody-cytolysin conjugate and secondly the ICI. Alternatively, the method may comprise sequential administration of first the ICI and secondly the antibody-cytolysin conjugate.

In some embodiments, the method comprises intravenous administration of the antibody-cytolysin conjugate and the ICI to the subject. In such embodiments, it will be appreciated that each of the antibody-cytolysin conjugate and the ICI are administered intravenously, sequentially, simultaneously or separately, preferably sequentially.

In some embodiments, the antibody-cytolysin conjugate is administered weekly. In some embodiments, the antibody-cytolysin conjugate is administered every two weeks. Two weeks may otherwise be referred to as 14 days. In some embodiments, the antibody-cytolysin conjugate is administered every three weeks. Three weeks may otherwise be referred to as 21 days. In some embodiments, the antibody-cytolysin conjugate is administered monthly.

In some embodiments, the method comprises administration of the antibody-cytolysin conjugate at least once in a 21-day cycle. In some embodiments, the method comprises administration of the antibody-cytolysin conjugate at least twice in a 21-day cycle.

In some embodiments, the method comprises administration of the antibody-cytolysin conjugate only once in a 21-day cycle. In some embodiments, the method comprises administration of the antibody-cytolysin conjugate only twice in a 21-day cycle.

In some embodiments, the method comprises administration of the antibody-cytolysin conjugate on day 1 and day 8 of a 21-day cycle. "Day 1" will be understood to refer to the first day of the 21-day cycle.

In embodiments comprising 21-day cycles, in some embodiments the antibody-cytolysin conjugate is not administered on days 9 to 21 of the 21-day cycle.

The ICI may be administered on the same day as the antibody-cytolysin conjugate. In some embodiments, the ICI is administered weekly. In some embodiments, the ICI is administered every two weeks. In some embodiments, the ICI is administered every three weeks. In some embodiments, the ICI is administered monthly.

In some embodiments, the method comprises administration of the ICI at least once in a 21-day cycle. The method may comprise administration of the ICI on day 1 of a 21-day cycle.

In some embodiments, the method comprises administration of the ICI only once in a 21-day cycle.

In some embodiments, the method comprises administration of the antibody-cytolysin conjugate and the ICI on day 1 of a 21-day cycle.

In some embodiments, the method comprises administration of:
the antibody-cytolysin conjugate and the ICI on day 1 of a 21day cycle; and
the antibody cytolysin conjugate on day 8 of a 21-day cycle.

In embodiments comprising a 21-day cycle of administration, the 21-day cycle may be repeated at least once. For example, the method may comprise administration of the antibody-cytolysin conjugate and the ICI to the subject in at least two, at least three, at least four, at least five, at least six, at least seven, at least eight, at least nine or at least 10 21-day cycles. In some embodiments, the method comprises administration of the antibody-cytolysin conjugate and the ICI in at least 10 21-day cycles. Each 21-day cycle may comprise the same or different administration pattern as described above.

For example, each 21-day cycle may comprise administration of the antibody-cytolysin conjugate on day 1 of each 21-day cycle. In some embodiments, each 21-day cycle comprises administration of the antibody-cytolysin conjugate on day 1 and day 8 of each 21 day cycle. Each 21-day cycle may comprise administration of the ICI of day 1 of each 21-day cycle.

In some embodiments, the method comprises administration of at least 1 mg/kg of the antibody-cytolysin conjugate to the subject, optionally 2mg/kg of the antibody-cytolysin conjugate to the subject, further optionally at least 4mg/kg of the antibody-cytolysin conjugate to the subject. In some embodiments, the method comprises administration of at least 3mg/kg of the antibody-cytolysin conjugate to the subject. In some embodiments, the method comprises administration of at least 5mg/kg of the antibody-cytolysin conjugate to the subject. In some embodiments, the method comprises administration of at least 7mg/kg of the antibody-cytolysin conjugate to the subject. In some embodiments, the method comprises administration of at least 8mg/kg of the antibody-cytolysin conjugate to the subject. In some embodiments, the method comprises administration of at least 10mg/kg of the antibody-cytolysin conjugate to the subject. In some embodiments, the method comprises administration of from 2mg/kg to 10mg/kg of the antibody-cytolysin conjugate to the subject. In some embodiments, the method comprises administration of from 2mg/kg to 8mg/kg of the antibody-cytolysin conjugate to the subject. In some embodiments, the method comprises administration of from 2mg/kg to 6mg/kg of the antibody-cytolysin conjugate to the subject, optionally of from 2mg/kg to 5.5mg/kg In some embodiments, the method comprises administration of from 2mg/kg to 18mg/kg of the antibody-cytolysin conjugate to the subject. In some embodiments, the method comprises administration of from 4mg/kg to 10mg/kg of the antibody-cytolysin conjugate to the subject. In some embodiments, the method comprises administration of from 4mg/kg to 8mg/kg of the antibody-cytolysin conjugate to the subject. Optionally, the method comprises administration of from 4mg/kg to 7.5mg/kg of the antibody-cytolysin conjugate to the subject. In some embodiments, the method comprises administration of from 4mg/kg to 5.5mg/kg of the antibody-cytolysin conjugate to the subject.

In some embodiments, the method comprises administration of no more than 10mg/kg, optionally no more than 7.5mg/kg, no more than 6mg/kg or no more than 5mg/kg of the antibody-cytolysin conjugate to the subject. In some embodiments, the method comprises administration of no more than 5.5mg/kg of the antibody-cytolysin conjugate to the subject.

In some embodiments, the method comprises administration of from 2mg/kg to 4mg/kg of the antibody-cytolysin conjugate to the subject.

In some embodiments, the method comprises administration of about 4mg/kg of the antibody-cytolysin conjugate to the subject. In some embodiments, the method comprises administration of about 5.5mg/kg of the antibody-cytolysin conjugate to the subject. In some embodiments the method comprises administration of about 7.5mg/kg of the antibody-cytolysin conjugate to the subject.

Preferably, the mg/kg dosage selected will be understood to be the mg/kg administered per administration. Thus, for example, in embodiments where the antibody-cytolysin conjugate is administered at least twice, the mg/kg dosage may be as described above for each administration.

Thus, in some embodiments, the method comprises administration of the antibody-cytolysin conjugate at least twice in a 21-day cycle, wherein each administration comprises at least 1 mg/kg, optionally at least 2mg/kg of the antibody-cytolysin conjugate to the subject.

In some embodiments, the method comprises administration of the antibody-cytolysin conjugate at least twice in a 21-day cycle, wherein each administration comprises at least 4mg/kg, optionally at least 7mg/kg of the antibody-cytolysin conjugate to the subject. In some embodiments, the method comprises administration of the antibody-cytolysin conjugate at least twice in a 21-day cycle, wherein each administration comprises at least 7.5mg/kg, optionally at least 10mg/kg of the antibody-cytolysin conjugate to the subject.

In some embodiments, the method comprises administration of at least 100mg, at least 150mg, at least 200mg, at least 250mg, at least 300mg, at least 350mg or at least 400mg of the ICI to the subject. In some embodiments, the method comprises administration of from 100mg to 500mg of the ICI to the subject. In some embodiments, the method comprises administration of from 100mg to 400mg of the ICI to the subject, optionally of from 100mg to 300mg of the ICI to the subject. In some embodiments, the method comprises administration of at least 200mg of the ICI to the subject. In some embodiments, the method comprises administration of 200mg of ICI to the subject. The mg of ICI selected for administration may be per administration (for example 100mg per administration in embodiments where the ICI is administered twice). Preferably, the mg of ICI selected may be the total mg of ICI administered per treatment cycle.

Thus, in some embodiments, the method comprises administration of the ICI once in a 21-day cycle, wherein the administration comprises administration of 200mg of ICI to the subject.

### Pharmaceutical compositions

The antibody-cytolysin conjugate and/or ICI of the present invention may be comprised in pharmaceutical compositions with a pharmaceutically acceptable excipient.

A pharmaceutically acceptable excipient may be a compound or a combination of compounds entering into a pharmaceutical composition which does not provoke secondary reactions and which allows, for example, facilitation of the administration of the conjugate/ICI, an increase in its lifespan and/or in its efficacy in the body or an increase in its solubility in solution. These pharmaceutically acceptable vehicles are well known and will be adapted by the person skilled in the art as a function of the mode of administration of the conjugate/ICI.

In some embodiments, conjugates and/or ICls of the present invention may be provided in a lyophilised form for reconstitution prior to administration. For example, lyophilised conjugates may be re-constituted in sterile water and mixed with saline prior to administration to an individual.

Conjugates and/or ICls of the present invention may be administered in the form of a pharmaceutical composition, which may comprise at least one component in addition to the conjugate. Thus pharmaceutical compositions may comprise, in addition to the conjugate, a pharmaceutically acceptable excipient, carrier, buffer, stabilizer or other materials well known to those skilled in the art. Such materials should be non-toxic and should not interfere with the efficacy of the conjugate/ICI. The precise nature of the carrier or other material will depend on the route of administration, which may be by bolus, infusion, injection or any other suitable route, as discussed below.

For intra-venous administration, for example by injection, the pharmaceutical composition comprising the conjugate and/or ICI may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or non-ionic surfactants, such as TWEEN^{™}, PLURONICS^{™} or polyethylene glycol (PEG).

### Method of treatment

According to a second aspect, the present invention provides a method of treating metastatic cancer in a mammalian subject, the method comprising administering (i) an anti-Fibroblast Activating Protein α (FAP) antibody-cytolysin conjugate having the formula A-(L-D)ₚ or a pharmaceutically acceptable salt or solvate thereof, wherein A is an anti-FAP antibody that selectively binds FAP, L is a linker, D is a drug comprising a cytolysin and p is 1 to 10, to the subject simultaneously, sequentially or separately with (ii) an immune checkpoint inhibitor (ICI), wherein the metastatic cancer comprises a primary cancer and metastasis.

The primary cancer of the metastatic cancer may comprise lung or gastrointestinal cancer. It will be appreciated that the method comprises administration of a therapeutically effective amount of the antibody-cytolysin conjugate and ICI as defined in accordance with the first aspect of the invention to the subject in need thereof. Any feature of the first aspect equally applies to the second aspect.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1: PHASE 1 DOSE-ESCALATION TRIAL OF OMTX705 AS A SINGLE AGENT AND IN COMBINATION WITH AN IMMUNE CHECKPOINT INHIBITOR (ICI)

OMTX705, which is described in WO 2024/023159, is an anti-Fibroblast Activation Protein α (FAP)-targeted antibody-drug conjugate, wherein the antibody is conjugated to a drug comprising cytolysin. We decided to investigate the safety and preliminary anti-tumour activity of this antibody as a monotherapy and in combination with anti-PD1 immune checkpoint inhibitors in subjects with solid tumours, where the cancer was metastatic. For such subjects, there is no available standard therapeutic option.

The study was an open-label, two parallel arm, multicenter, Phase 1 dose-escalation study designed to evaluate the safety, tolerability and preliminary antitumour activity of OMTX705, both as a single agent (monotherapy) or in combination with the anti-PD-1 inhibitor pembrolizumab. The trial enrolled selected tumour indications that are known to express FAP either on tumour stroma CAFs (carcinomas) or on tumour cells (leiomyosarcomas and other sarcomas).

The Phase 1 dose-escalation was carried out with two parallel staggered escalation cohorts. One cohort of subjects was treated with OMTX705 as a monotherapy and one cohort of subjects received escalating doses of OMTX705 in combination with a standard dose (200mg) of pembrolizumab.

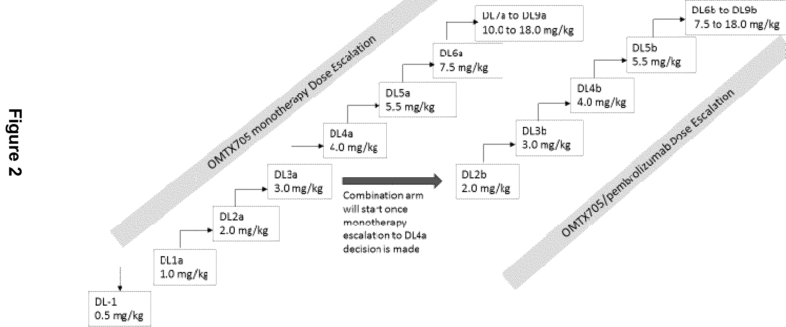

The combination arm started once dose level (DL) 3a OMTX705 (3.0 mg/kg) in monotherapy was confirmed as safe. The starting dose of OMTX705 for the combination was the same as monotherapy DL2a (2.0 mg/kg). A schematic of the dose-escalation scheme is shown in Figure 2. For the combination scheme, both OMTX705 and pembrolizumab were administered on day 1 of a 21-day cycle. A second dose of OMTX705 was administered in the same cycle, on day 8. However, pembrolizumab was only administered once in each cycle.

64 subjects received OMTX705: 27 in monotherapy and 37 in combination with pembrolizumab. In the monotherapy cohort, 10 subjects had primary sarcomas and 17 subjects had primary carcinomas. The carcinomas included 2 colorectal, 5 pancreatic, 6 non-small cell lung, 1 gastric, 2 ovary and 1 breast cancer.

In the combination cohort, all 37 subjects had primary carcinomas, including 12 colorectal, 14 pancreatic, 3 oesophageal, 1 cholangiocarcinoma, 3 non-small cell lung, 3 lung mesothelioma and 1 head and neck cancer.

All subjects had metastatic cancer. The multiple anatomical locations of the cancer of each subject in the monotherapy cohort is shown in Table 1 and in Table 2 for the combination cohort.

**Table 1: Primary cancer type and tumour location for subjects in monotherapy cohort.**

| **Subject ID** | **Tumour location** | **Primary tumour type** | |
|---|---|---|---|
| **01-002** | Liver | **Carcinoma** | CRC |
| **04-001** | Pancreas, liver, locoregional lymph nodes (LN) | **Carcinoma** | Pancreatic cancer |
| **03-002** | Lung | **Sarcoma** | Leiomyosarcoma (LMS) |
| **04-002** | Liver, mesenterium, paravertebral muscle, iliac bone, lung lingula | **Sarcoma** | LMS |
| **02-001** | Pancreas, liver | **Carcinoma** | PDAC |
| **03-004** | Lung, bone, LN (liver, mediastinum, hiliar), pleura, peritoneum | **Carcinoma** | NSCLC |
| **01-003** | Bilateral lung, stomach, peritoneal carcinomatosis | **Carcinoma** | GASTRIC |
| **02-003** | Lung, subcutaneous breast, chest soft tissue, liver, adrenal, pancreas, bilateral kidney | **Sarcoma** | LMS |
| **04-003** | Pleura, peritoneum | **Carcinoma** | High-grade serious ovarian cancer (HGSOC) |
| **01-004** | Omentum (stomach), pancreas, lung | **Carcinoma** | PDAC |
| **11-002** | Both ovaries, multiple LNs, abdominal wall, peritoneum, pleura, chest wall. | **Carcinoma** | HGSOC |
| **02-005** | Liver (multiple), lung (multiple), pleural effusion, ascites | **Sarcoma** | LMS |
| **01-005** | Right hemipelvis, obturator muscle, chest, breast | **Sarcoma** | LMS |
| **03-007** | Liver (multiple), adrenal | **Sarcoma** | LMS |
| **03-008** | Lung, LN (multiple), liver, pleura, bone | **Carcinoma** | NSCLC |
| **01-007** | Muscle (multiple), lung (multiple) | **Sarcoma** | LMS |
| **04-007** | Lung (multiple),LN (mediastinal retroperitoneal, supraclavicular), bone (L1 and D8) | **Carcinoma** | CRC |
| **07-003** | Liver (multiple), lung (multiple), bone | **Carcinoma** | Breast cancer (BC) |
| **11-007** | Pancreas, liver (multiple), LN (abdominal and thoracic), peritoneum, lung | **Carcinoma** | PDAC |
| **03-012** | Lung (multiple), liver, brain | **Carcinoma** | NSCLC |
| **03-013** | Lung, soft tissue, subcarinal LN, mediastinal LN | **Carcinoma** | NSCLC |
| **11-009** | LNs (multiple both sides of diaphragm), lung, liver (multiple) | **Carcinoma** | NSCLC |
| **04-011** | Lung, subhepatic | **Sarcoma** | Osteosarcoma |
| **02-013** | Liver | **Carcinoma** | PDAC |
| **11-011** | Pleura, LNs, pelvic peritoneum, abdominal peritoneum and omentum, subcutaneous, bone | **Sarcoma** | Endometrial stromal sarcoma |
| **03-018** | Lung, liver, LN (multiple) | **Carcinoma** | NSCLC |
| **03-019** | Peritoneum, stomach, liver, soft tissue | **Sarcoma** | Liposarcoma |

**Table 2: Primary cancer type and tumour location for subjects in combination therapy cohort.**

| **Subject ID** | **Tumour location** | **Primary tumour type** |
|---|---|---|
| **04-004** | Lung, liver lymph nodes, scrotum | CRC |
| **11-001** | Abdominal wall, peritoneum/omentum, lung (multiple), sigmoid colon wall | CRC |
| **03-006** | Lung, LNs: subcarinal, upper abdomen, | NSCLC |
| **02-006** | Liver (multiple), LN (multiple) | Esophageal |
| **04-005** | Lung, head and neck, LNs | Head and neck squamous cell carcinoma (HNSCC) |
| **04-006** | Esophagus, bone, LN (hilium) | Esophageal |
| **11-004** | Peritoneum, bile duct, presacral soft tissue | CRC |
| **07-001** | Lung (multiple) | PDAC |
| **07-002** | Pancreas, soft tissue left external iliac, hepatic and peripancreatic | PDAC |
| **11-005** | Esophagus, lung (multiple) | Esophageal |
| **03-011** | Pancreas soft tissue, mass adjacent to stomach, peritoneum | PDAC |
| **07-005** | Lung and thoracic LNs | NSCLC |
| **11-008** | Liver (multiple) | CRC |
| **07-007** | Mediastinum, SC LNs, pleural, chest wall, pericardium, iliacus and psoas | Mesothelioma |
| **03-015** | Lung, mediastinal LN | NSCLC |
| **03-016** | Lung, pleural, mediastinal LN | Mesothelioma |
| **03-017** | Soft tissue, pleura, LNs (multiple), liver, kidney, lungs, mediastinum, pericardial effusion, peritoneal, bone | Mesothelioma |
| **04-014** | Mediastinal LN (multiple), lung, bone | Cholangiocarcinoma |
| **07-004** | Lung (multiple, bilateral), supraclavicular and retroperitoneal LN, pancreatic tail, liver (multiple) | PDAC |
| **11-006** | Pleura, LN (mediastinum, SC, retroperitoneal, hilum), lung, adrenal | CRC |
| **02-011** | Liver, pancreas | PDAC |
| **03-014** | Liver (multiple) | CRC |
| **04-008** | Liver (multiple), LN (multiple), pancreas | CRC |
| **04-009** | Pancreas, LN, peritoneum, pleura | PDAC |
| **04-010** | Lymph nodes interaortocaval and paraaortic, omental implants | CRC |
| **07-006** | Lung, liver, periportal LN, mesenteric LN, pouch of Douglas, peritoneal carcinomatosis | PDAC |
| **02-012** | Liver, pancreas, retroperitoneal LN | PDAC |
| **11-010** | Lung, pancreas, peritoneum | PDAC |
| **04-012** | Liver, LN (multiple) | CRC |
| **07-008** | LN iliac and retroperitoneal | CRC |
| **02-018** | Liver, adrenal | PDAC |
| **04-013** | Lung, mediastinal LN | CRC |
| **02-015** | Lung | CRC |
| **07-009** | Liver and lung | PDAC |
| **01-010** | Liver and lung | PDAC |
| **04-015** | Pancreas, lung, LN | PDAC |
| **01-012** | Pancreas, liver, peritoneum, LN, lung, adrenal | PDAC |

No dose-limiting toxicity was observed in any of the monotherapy or combination subjects. Nor did any subject require an OMTX705 dose reduction. Dose escalation in the combination was halted at 10 mg/kg of OMTX705, because it was considered that in the absence of Dose-Limiting Toxicities (DLTs), the antitumour activity did not further increase when increasing the dose higher than 4 mg/kg (5.5, 7.5 and 10 mg/kg).

Turning to efficacy, for the monotherapy cohort, 27 subjects were dosed and have efficacy information. The monotherapy did not provide any significant anti-tumour activity in the subjects with carcinomas; 16/17 carcinoma subjects had Progressive Disease (PD) as the best response, with the sole exception of a pancreatic cancer subject who had stable disease for only 4 cycles. Four of the 10 sarcoma subjects had stable disease as the best response. The remaining 6 sarcoma subjects had Progressive Disease (PD) as the best response. Progressive Disease (PD) was determined as the response when the sum of diameters or number of tumour lesions was 20% higher than the previous lowest value for the sum of diameters/number of tumour lesions. These results are shown in Tables 3 and 4, below. Disease control rate (DCR) will be understood to mean the combination of partial responses and stable disease.

**Table 3: Tumour responses (as defined per RECIST1.1) of carcinoma monotherapy cohort (N=17).**

| **Carcinoma OMTX705 Monotherapy** | |
|---|---|
| **Tumour responses** | **Number of cohort (%)** |
| Partial Responses (PRs, defined as >30% tumour size reduction) | 0/17 |
| Stable Disease (SD) | 1/17 (0.6%) |
| Disease Control Rate (DCR) | 1/17 (0.6%) |

**Table 4: Tumour responses (as defined per RECIST1.1) of sarcoma monotherapy cohort (N=10).**

| **Sarcoma OMTX705 Monotherapy** | |
|---|---|
| **Tumour responses** | **Number of cohort (%)** |
| Partial Responses (PRs, defined as >30% tumour size reduction) | 0/17 |
| Stable Disease (SD) | 4/10 (40%) |
| Disease Control Rate (DCR) | 4/10 (40%) |

For the combination cohort, 37 subjects were dosed and have efficacy information. 12 had colorectal cancer, 14 had PDAC, three had non-small cell lung cancer (NSCLC), three had oesophageal cancer, 1 had cholangiocarcinoma, three had lung mesothelioma and one had head and neck squamous cell carcinoma (HNSCC). All 12 of the colorectal cancer subjects were identified as microsatellite-stable (MSS) colorectal cancer using routine methods known in the art, and so considered non-eligible for mono-immunotherapy. One subject had received previous anti-PD1 immunotherapy. All 14 of the PDAC subjects were considered to be Mismatch Repair Proficient and so considered non-eligible for mono-immunotherapy. One had received previous anti-PDL1 and anti-CTLA4 combined immunotherapy. All 3 NSCLC subjects had previously received immunotherapy.

Of the 37 combination therapy subjects, 18 had Progressive Disease (PD) as the best response. Table 5, below, shows the number (and percentage) of the entire combination cohort who had a tumour response (i.e. not progressive disease) to the combination therapy.

**Table 5: Tumour responses (as defined per RECIST1.1) of combination therapy cohort (N=37).**

| **Combination (OMTX705 + ICI) Therapy** | |
|---|---|
| **Tumour responses** | **Number of cohort (%)** |
| Partial Responses (PRs, defined as >30% tumour size reduction) | 2/37 confirmed PRs at 4 mg/kg (escalation) (5%): 1 in CRC MSS with liver metastases and 1 in PDAC. Long duration of Partial Responses: > 15 cycles. |
| Stable Disease (SD) | 17/37 (46%) |
| Disease Control Rate (DCR) | 19/37 (51%) |

Overall, the best response to the combination therapy was achieved in subjects with PDAC or CRC, with around 50% of subjects achieving a partial response or stable disease (see Tables 6 and 7).

The specific number of cycles, days thereof and response for each subject is shown for PDAC and CRC subjects in Tables 8 and 9, respectively.

**Table 6: Tumour responses (as defined per RECIST1.1) of combination therapy PDAC cohort (N=14).**

| **PDAC Combination (OMTX705 + ICI) Therapy** | |
|---|---|
| **Tumour responses** | **Number of cohort (%)** |
| Partial Responses (PRs, defined as >30% tumour size reduction) | 1/14 at 4 mg/kg (escalation) (7%). |
| Stable Disease (SD) | 7/14 (50%) |
| Disease Control Rate (DCR) | 8/14 (57%) |

**Table 7: Tumour responses (as defined per RECIST1.1) of combination therapy CRC cohort (N=12).**

| **CRC Combination (OMTX705 + ICI) Therapy** | |
|---|---|
| **Tumour responses** | **Number of cohort (%)** |
| Partial Responses (PRs, defined as >30% tumour size reduction) | 1/12 confirmed PRs at 4 mg/kg (escalation) (8%). |
| Stable Disease (SD) | 4/12 (33%) |
| Disease Control Rate (DCR) | 5/12 (42%) |

**Table 8: Cycle and response details for each PDAC subject in the combination therapy cohort. Tumour responses defined per RECIST1.1. Partial response (PR) was defined as >30% tumour size reduction. Stable disease (SD) was defined as a tumour size reduction of between ≥0 and ≤30%. Progressive disease (PD) was defined as an increase in tumour size or where trial ended early for that subject. "iuPD" refers to unconfirmed progressive disease. L refers to the dose of OMTX705 administered to the subject, as defined in Figure 2. L2 = 2mg/kg, L3 = 3mg/kg, L4 = 4mg/kg, L5 = 5.5mg/kg, L6= 7.5mg/kg. For each cycle, a standard dose (200mg) of pembrolizumab was administered on day 1 of the cycle.**

| **Subject** | **No. of Completed Cycles** | **Days in trial (typically 21 days per cycle)** | **Months in trial** | **Response %** | **Respons e class** | **L** |
|---|---|---|---|---|---|---|
| 03-011 | 1 | 21 | 1 | NA | PD | 2 |
| 04-009 | 1 | 21 | 1 | NA | PD | 6 |
| 02-018 | 2 | 42 | 1 | 50 | PD | 2 |
| 07-004 | 2 | 42 | 1 | -32 | SD | 3 |
| 07-006 | 2 | 43 | 1 | 25 | PD | 3 |
| 01-010 | 2 | 43 | 1 | 40 | PD | 3 |
| 04-015 | 2 | 46 | 2 | 0 | SD | 3 |
| 01-012 | 3 | 53 | 2 | -10 | SD | 4 |
| 07-009 | 4 | 82 | 3 | 8 | SD | 4 |
| 02-012 | 4 | 84 | 3 | -11 | iuPD | 3 |
| 11-010 | 6 | 125 | 4 | -14 | SD | 3 |
| 02-011 | 9 | 182 | 6 | -4 | SD | 3 |
| 07-002 | 13 | 283 | 9 | -18 | SD | 4 |
| 07-001 | 15 | 307 | 10 | -46 | PR | 4 |

**Table 9: Cycle and response details for each CRC subject in the combination therapy cohort. Tumour responses defined per RECIST1.1. Partial response (PR) was defined as >30% tumour size reduction. Stable disease (SD) was defined as a tumour size reduction of between ≥0 and ≤30%. Progressive disease (PD) was defined as an increase in tumour size or where trial ended early for that subject. L refers to the dose of OMTX705 administered to the subject, as defined in Figure 2. L2 = 2mg/kg, L3 = 3mg/kg, L4 = 4mg/kg, L5 = 5.5mg/kg, L6= 7.5mg/kg. For each cycle, a standard dose (200mg) of pembrolizumab was administered on day 1 of the cycle.**

| **Subject** | **No. of Completed Cycles** | **Days in trial (typically 21 days per cycle)** | **Months in trial** | **Response %** | **Response class** | **L** | **Liver metastases** |
|---|---|---|---|---|---|---|---|
| 03-014 | 0 | 8 | 0 | 0 | PD | 6 | Y |
| 11-006 | 1 | 22 | 1 | 13 | PD | 5 | N |
| 04-010 | 1 | 29 | 1 | 22 | PD | 2 | N |
| 04-008 | 1 | 29 | 1 | 0 | PD | 5 | Y |
| 11-008 | 1 | 29 | 1 | 43 | PD | 3 | Y |
| 02-015 | 4 | 84 | 3 | 26 | PD | 6 | N |
| 04-013 | 4 | 84 | 3 | 35 | PD | 5 | N |
| 04-012 | 4 | 84 | 3 | 0 | SD | 3 | Y |
| 07-008 | 6 | 126 | 4 | -14 | SD | 3 | peritoneal |
| 11-001 | 6 | 134 | 4 | 5 | SD | 4 | peritoneal |
| 04-004 | 12 | 252 | 8 | -24 | SD | 5 | y |
| 11-004 | 15 | 319 | 11 | -43 | PR | 2 | Y |

As Table 9 shows, of the five CRC subjects in the combination cohort who responded to treatment, three of the subjects had liver metastases and two subjects had peritoneal metastases.

The best response was achieved in cycles where at least 4mg/kg of OMTX705 was administered in combination with pembrolizumab, although tumour response was also seen at lower concentrations of OMTX705, such as at 3mg/kg (see Tables 8 and 9).

Promising responses were also achieved in NSCLC and oesophageal cancer. Although no partial response was achieved for NSCLC or oesophageal cancer, at least two thirds of subjects exhibited stable disease when treated with the combination therapy. Indeed, for NSCLC subjects, 100% of the combination cohort exhibited stable disease. These results are shown in Tables 10 and 11.

**Table 10: Tumour responses (as defined per RECIST1.1) of combination therapy NSCLC cohort (N=3).**

| **NSCLC Combination (OMTX705 + ICI) Therapy** | |
|---|---|
| **Tumour responses** | **Number of cohort (%)** |
| Partial Responses (PRs, defined as >30% tumour size reduction) | 0/3 (0%) |
| Stable Disease (SD) | 3/3 (100%) |
| Disease Control Rate (DCR) | 3/3 (100%) |

**Table 11: Tumour responses (as defined per RECIST1.1) of combination therapy oesophageal cancer cohort (N=3).**

| **Oesophageal Cancer Combination (OMTX705 + ICI) Therapy** | |
|---|---|
| **Tumour responses** | **Number of cohort (%)** |
| Partial Responses (PRs, defined as >30% tumour size reduction) | 0/3 (0%) |
| Stable Disease (SD) | 2/3 (67%) |
| Disease Control Rate (DCR) | 2/3 (67%) |

### Sequences

In the following sequences, VH and VL domains are underlined and CDRH/CDRL regions are in bold. Mutations leading to ADCC and CDC deficiency are shown in bold italics. Signal sequences (where applicable) are shown boxed.
*hu36 IgG1-HC - with signal sequence* 
METDTLLLWVLLLWVPGSTG
QVQLVQSGAEVKKPGASVKVSCKASGYTFTENIIHWVRQAPGQGLEWMGWFHPGSGSIKYNEKFKDRV
TMTADTSTSTVYMELSSLRSEDTAVYYCARHGGTGRGAMDYWGQGTLVTVSSASTKGPSVFPLAPSSK
STSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPPVAGPSVFLFPPKPKDTLMISRTPEVTCVVVDVSHEDPEVK
FNWYVDGVEVHNAKTKPREEQYNSTYRVVSVLTVLHQDWLNGKEYKCKVSNKGLPSSIEKTISKAKGQP
REPQVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 1)
*hu36-IgG1-LC - with signal sequence:* 
METDTLLLWVLLLWVPGSTG
DIQMTQSPSSLSASVGDRVTITCRASKSVSTSAYSYMHWYQQKPGKAPKLLIYLASNLESGVPSRFSGS
GSGTDFTLTISSLQPEDFATYYCQHSRELPYTFGQGTKLEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC (SEQ ID NO: 2)
*hu36-IgG1-HC - without signal sequence:* 
QVQLVQSGAEVKKPGASVKVSCKASGYTFTENIIHWVRQAPGQGLEWMGWFHPGSGSIKYNEKFKDRV
TMTADTSTSTVYMELSSLRSEDTAVYYCARHGGTGRGAMDYWGQGTLVTVSSASTKGPSVFPLAPSSK
STSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPPVAGPSVFLFPPKPKDTLMISRTPEVTCVVVDVSHEDPEVK
FNWYVDGVEVHNAKTKPREEQYNSTYRVVSVLTVLHQDWLNGKEYKCKVSNKGLPSSIEKTISKAKGQP
REPQVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 3)
*hu36-IgG1-LC - without signal sequence:* 
DIQMTQSPSSLSASVGDRVTITCRASKSVSTSAYSYMHWYQQKPGKAPKLLIYLASNLESGVPSRFSGS
GSGTDFTLTISSLQPEDFATYYCQHSRELPYTFGQGTKLEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC (SEQ ID NO: 4)
*hu36-VH:* 
QVQLVQSGAEVKKPGASVKVSCKASGYTFTENIIHWVRQAPGQGLEWMGWFHPGSGSIKYNEKFKDRV
TMTADTSTSTVYMELSSLRSEDTAVYYCARHGGTGRGAMDYWGQGTLVTVSS (SEQ ID NO: 5)
*hu36-VL:* 
DIQMTQSPSSLSASVGDRVTITCRASKSVSTSAYSYMHWYQQKPGKAPKLLIYLASNLESGVPSRFSGS
GSGTDFTLTISSLQPEDFATYYCQHSRELPYTFGQGTKLEIKR (SEQ ID NO: 6)
*hu36-CDRH1:*
   ENIIH (SEQ ID NO: 7)
*hu36-CDRH2:* WFHPGSGSIKYNEKFKD (SEQ ID NO: 8)
*hu36-CDRH3:* HGGTGRGAMDY (SEQ ID NO: 9)
*hu36-CDRL1:* RASKSVSTSAYSYMH (SEQ ID NO: 10)
*hu36-CDRL2:* LASNLES (SEQ ID NO: 11)
*hu36-CDRL3:* QHSRELPYT (SEQ ID NO: 12)
*Human FAP*
Also known as Seprase, 170 kDa melanoma membrane-bound gelatinase, fibroblast activation protein alpha or integral membrane serine protease. The amino acid sequence is disclosed at UniProt accession No. Q12884 (Version 140, dated 11 December 2013): 
MKTWVKIVFGVATSAVLALLVMCIVLRPSRVHNSEENTMRALTLKDILNGTFSYKTFFPNWISGQEYLHQS
ADNNIVLYNIETGQSYTILSNRTMKSVNASNYGLSPDRQFVYLESDYSKLWRYSYTATYYIYDLSNGEFVR
GNELPRPIQYLCWSPVGSKLAYVYQNNIYLKQRPGDPPFQITFNGRENKIFNGIPDWVYEEEMLATKYAL
WWSPNGKFLAYAEFNDTDIPVIAYSYYGDEQYPRTINIPYPKAGAKNPVVRIFIIDTTYPAYVGPQEVPVPA
 
MIASSDYYFSWLTWVTDERVCLQWLKRVQNVSVLSICDFREDWQTWDCPKTQEHIEESRTGWAGGFFV
STPVFSYDAISYYKIFSDKDGYKHIHYIKDTVENAIQITSGKWEAINIFRVTQDSLFYSSNEFEEYPGRRNIY
RISIGSYPPSKKCVTCHLRKERCQYYTASFSDYAKYYALVCYGPGIPISTLHDGRTDQEIKILEENKELENA
LKNIQLPKEEIKKLEVDEITLWYKMILPPQFDRSKKYPLLIQVYGGPCSQSVRSVFAVNWISYLASKEGMVI
ALVDGRGTAFQGDKLLYAVYRKLGVYEVEDQITAVRKFIEMGFIDEKRIAIWGWSYGGYVSSLALASGTG
LFKCGIAVAPVSSWEYYASVYTERFMGLPTKDDNLEHYKNSTVMARAEYFRNVDYLLIHGTADDNVHFQ
NSAQIAKALVNAQVDFQAMWYSDQNHGLSGLSTNHLYTHMTHFLKQCFSLSD (SEQ ID NO: 13)
*Murine FAP*

Also known as fibroblast activation protein alpha or integral membrane serine protease. Amino acid
sequence is disclosed at UniProt accession No. P97321 (Version 117, dated 11 December 2013):
MKTWLKTVFGVTTLAALALVVICIVLRPSRVYKPEGNTKRALTLKDILNGTFSYKTYFPNWISEQEYLHQSE
DDNIVFYNIETRESYIILSNSTMKSVNATDYGLSPDRQFVYLESDYSKLWRYSYTATYYIYDLQNGEFVRG
YELPRPIQYLCWSPVGSKLAYVYQNNIYLKQRPGDPPFQITYTGRENRIFNGIPDWVYEEEMLATKYALW
WSPDGKFLAYVEFNDSDIPIIAYSYYGDGQYPRTINIPYPKAGAKNPVVRVFIVDTTYPHHVGPMEVPVPE
MIASSDYYFSWLTWVSSERVCLQWLKRVQNVSVLSICDFREDWHAWECPKNQEHVEESRTGWAGGFF
VSTPAFSQDATSYYKIFSDKDGYKHIHYIKDTVENAIQITSGKWEAIYIFRVTQDSLFYSSNEFEGYPGRRNI
YRISIGNSPPSKKCVTCHLRKERCQYYTASFSYKAKYYALVCYGPGLPISTLHDGRTDQEIQVLEENKELE
NSLRNIQLPKVEIKKLKDGGLTFWYKMILPPQFDRSKKYPLLIQVYGGPCSQSVKSVFAVNWITYLASKEGI
VIALVDGRGTAFQGDKFLHAVYRKLGVYEVEDQLTAVRKFIEMGFIDEERIAIWGWSYGGYVSSLALASG
TGLFKCGIAVAPVSSWEYYASIYSERFMGLPTKDDNLEHYKNSTVMARAEYFRNVDYLLIHGTADDNVHF
QNSAQIAKALVNAQVDFQAMWYSDQNHGISSGRSQNHLYTHMTHFLKQCFSLSD (SEQ ID NO: 14)

### References

A number of publications are cited above in order to more fully describe and disclose the invention and the state of the art to which the invention pertains. Full citations for these references are provided below. The entirety of each of these references is incorporated herein.

Dung et al. PD-1 Blockade in Tumours with Mismatch-Repair Deficiency. (2015). New Engl. J. Med. 372:2509-2520. DOI: 10.1056/NEJMoa1500596

Eisenhauer EA, et al. New response evaluation criteria in solid tumours: revised RECIST guideline (version 1.1). Eur J Cancer. 2009 Jan;45(2):228-47. doi: 10.1016/j.ejca.2008.10.026. PMID: 19097774

Fabre, M. et al. OMTX705, a Novel FAP-Targeting ADC Demonstrates Activity in Chemotherapy and Pembrolizumab-Resistant Solid Tumor Models. (2020) doi:10.1158/1078-0432.CCR-19-2238.

Fukuoka et al. Regorafenib Plus Nivolumab in Patients With Advanced Gastric or Colorectal Cancer: An Open-Label, Dose-Escalation, and Dose-Expansion Phase Ib Trial (REGONIVO, EPOC1603). J Clin Oncol. 2020 Jun 20;38(18):2053-2061. doi: 10.1200/JCO.19.03296.

O'Reilly et al. Durvalumab with or without Tremelimumab for Patients with Metastatic Pancreatic Ductal Adenocarcinoma. (2019) JAMA Oncol. 5 (10): 1431-1438. doi: 10.1001/jamaoncol.2019.1588

Overman et al. Nivolumab in patients with metastatic DNA mismatch repair-deficient or microsatellite instability-high colorectal cancer (CheckMate 142): an open-label, multicentre, phase 2 study. Lancet Oncol. 2017 Sep;18(9):1182-1191. doi: 10.1016/S1470-2045(17)30422-9.

Renouf et al. The CCTG PA.7 phase II trial of gemcitabine and nab-paclitaxel with or without durvalumab and tremeliumumab as initial therapy in metastatic pancreatic ductal adenocarcinoma. (2022)Nat Commun. 13, 5020. https://doi.org/10.1038/s41467-022-32591-8

Sahin et al. Immunotherapy for Microsatellite Stable Colorectal Cancers: Challenges and Novel Therapeutic Avenues. Am Soc Clin Oncol Educ Book. 2022 Apr;42:1-12. doi: 10.1200/EDBK_349811. PMID: 35658496.

Seymour L et al. iRECIST: guidelines for response criteria for use in trials testing immunotherapeutics. Lancet Oncol. 2017 Mar;18(3):e143-e152. doi: 10.1016/S1470-2045(17)30074-8. Epub 2017 Mar 2. Erratum in: Lancet Oncol. 2019 May;20(5):e242. doi: 10.1016/S1470-2045(19)30240-2. PMID: 28271869; PMCID: PMC5648544.

Wainberg et al. Open-label, Phase I Study of Nivolumab combined with nab-Paclitaxel plus Gemcitabine in Advanced Pancreatic Cancer. (2020) Clin Cancer Res. 26(18): 4814-4822. DOI: 10.1158/1078-0432.CCR-20-0099

Wang et al. Clinical Response to Immunotherapy Targeting Programmed Cell Death Receptor 1/Programmed Cell Death Ligand 1 in Patients With Treatment-Resistant Microsatellite Stable Colorectal Cancer With and Without Liver Metastases. JAMA Netw Open. 2021;4(8):e2118416. doi:1 0.1 001/jamanetworkopen.2021.18416

Ye et al. Peritumoral Immune-suppressive Mechanisms Impede Intratumoral Lymphocyte Infiltration into Colorectal Cancer Liver versus Lung Metastases. Cancer Res Commun. 2023 Oct 12;3(10):2082-2095. doi: 10.1158/2767-9764.CRC-23-0212.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1. A combination of (i) an anti-Fibroblast Activating Protein α (FAP) antibody-cytolysin conjugate having the formula A-(L-D)ₚ or a pharmaceutically acceptable salt or solvate thereof, wherein A is an anti-FAP antibody that selectively binds FAP, L is a linker, D is a drug comprising a cytolysin and p is 1 to 10, and (ii) an immune checkpoint inhibitor (ICI) for use in a method of treating metastatic cancer in a mammalian subject, wherein the method comprises simultaneous, sequential or separate administration of the antibody-cytolysin conjugate or the pharmaceutically acceptable salt or solvate thereof and the ICI to the subject, wherein the metastatic cancer comprises a primary cancer and metastasis.

2. The combination for use of claim 1, wherein the primary cancer of the metastatic cancer comprises gastrointestinal cancer or lung cancer.

3. The combination for use of claim 1 or claim 2, wherein the ICI comprises an anti-PD-1 molecule, an anti-PD-L1 molecule or an anti-CTLA-4 molecule.

4. The combination for use of any one of claims 1 to 3, wherein the ICI comprises an anti-PD-1 molecule.

5. The combination for use of claim 3 or claim 4, wherein the anti-PD-1 molecule comprises pembrolizumab, nivolumab or tislelizumab .

6. The combination for use of any one of the preceding claims, wherein A of the antibody-cytolysin conjugate has a heavy chain with amino acid sequence of SEQ ID NO: 1 and a light chain with amino acid sequence of SEQ ID NO: 2.

7. The combination for use of any one of the preceding claims, wherein the cytolysin of the antibody-cytolysin conjugate comprises formula IV: 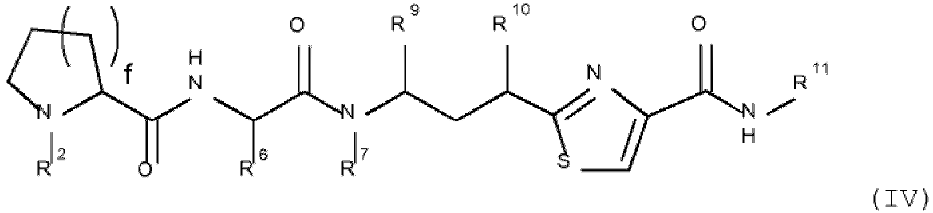 wherein:
R² is H or C₁-C₄ alkyl;
R⁶ is C₁-C₆ alkyl;
R⁷ is C₁-C₆ alkyl, CH₂OR¹⁹ or CH₂OCOR²⁰, wherein R¹⁹ is alkyl, R²⁰ is C₂-C₆-alkenyl, phenyl, or CH₂-phenyl;
R⁹ is C₁-C₆ alkyl;
R¹⁰ is H, OH, O-alkyl or O-acetyl;
f is 1 or 2;
R¹¹ has the following structure: 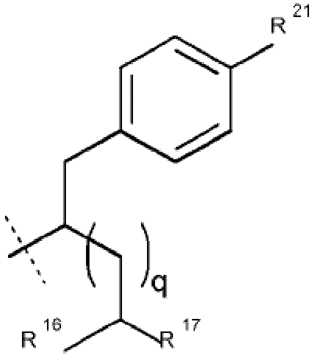
wherein
R²¹ is H, OH, halogen, NH₂, alkyloxy, phenyl, alkyl amino or dialkyl amino;
R¹⁶ is H or a C₁-C₆-alkyl group;
R¹⁷ is directly or indirectly attached to linker L; and
q is 0, 1, 2 or 3;
and wherein the term "optionally substituted" relates to groups, wherein one or several H atoms can be replaced by F, CI, Br or I or OH, SH, NH₂, or NO₂; the term "optionally substituted" further relates to groups, which can be exclusively or additionally substituted with unsubstituted C₁-C₆ alkyl, C₂C₆ alkenyl, C₂-C₆ alkynyl, C₁-C₆ heteroalkyl, C₃-C₁₀ cycloalkyl, C₂-C₉ heterocycloalkyl, C₆-C₁₀ aryl, C₁-C₉ heteroaryl, C₇-C₁₂ aralkyl or C₂-C11 heteroaralkyl groups.

8. The combination for use of any one of the preceding claims, wherein L of the antibody-cytolysin conjugate comprises a spacer, optionally wherein the spacer comprises -(OCH₂CH₂)ₙ-, wherein n is 2 to 5.

9. The combination for use of any one of the preceding claims, wherein L of the antibody-cytolysin conjugate comprises an attachment group for attachment to A; optionally wherein L comprises a protease cleavable portion comprising a valine-citrulline unit.

10. The combination for use of any one of the preceding claims, wherein the cytolysin of the antibody-cytolysin conjugate has the formula: 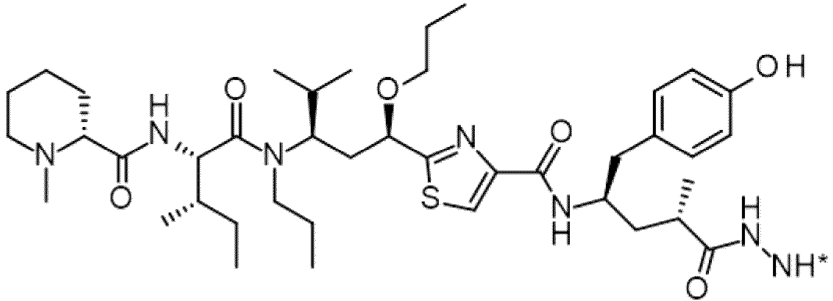 wherein * indicates the site of attachment to L.

11. The combination for use of any one of the preceding claims, wherein -L-D of the antibody-cytolysin conjugate has the structure: 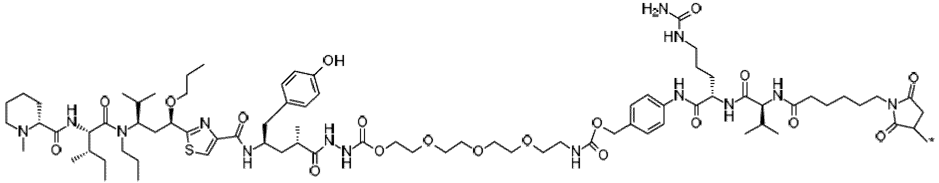 wherein * denotes the point of attachment to A.

12. The combination for use of any one of the preceding claims, wherein the mammalian subject is a human subject.

13. The combination for use of any one of the preceding claims, wherein the primary cancer comprises pancreatic cancer, colorectal cancer, oesophageal cancer, gastric (stomach) cancer, liver cancer or lung cancer.

14. The combination for use of any one of the preceding claims, wherein the primary cancer comprises pancreatic cancer, colorectal cancer, oesophageal cancer or lung cancer.

15. The combination for use of any one of the preceding claims, wherein the primary cancer comprises pancreatic or colorectal cancer.

16. The combination for use of any one of claims 13 to 15, wherein the pancreatic cancer comprises pancreatic ductal adenocarcinoma (PDAC).

17. The combination for use of any one of claims 13 to 15, wherein the colorectal cancer comprises microsatellite-stable (MSS) colorectal cancer.

18. The combination for use of any one of the preceding claims, wherein the primary cancer comprises MSS colorectal cancer and the metastasis comprises liver, lung and/or peritoneal metastasis.

19. The combination for use of any one of the preceding claims, wherein the subject was previously administered the antibody-cytolysin conjugate and was not previously administered the ICI.

20. The combination for use of any one of claims 1 to 18, wherein the subject was previously administered the ICI and was not previously administered the antibody-cytolysin conjugate.

21. The combination for use of any one of the preceding claims, wherein the method comprises sequential administration of the antibody-cytolysin conjugate and the ICI to the subject.

22. The combination for use of any one of the preceding claims, wherein the method comprises intravenous administration of the antibody-cytolysin conjugate and the ICI to the subject.

23. The combination for use of any one of the preceding claims, wherein the method comprises administration of the antibody-cytolysin conjugate at least once in a 21-day cycle.

24. The combination for use of any one of the preceding claims, wherein the method comprises administration of the antibody-cytolysin conjugate at least twice in a 21-day cycle.

25. The combination for use of claim 24, wherein the method comprises administration of the antibody-cytolysin conjugate on day 1 and day 8 of a 21-day cycle.

26. The combination for use of any one of the preceding claims, wherein the method comprises administration of the ICI at least once in a 21-day cycle.

27. The combination for use of claim 26, wherein the method comprises administration of the ICI on day 1 of a 21-day cycle.

28. The combination for use of any one of claims 23 to 27, wherein the 21-day cycle is repeated at least once.

29. The combination for use of any one of the preceding claims, wherein administration of the antibody-cytolysin conjugate and the ICI results in a synergistic effect.

30. The combination for use of any one of the preceding claims, wherein administration of the antibody-cytolysin conjugate and the ICI results in a reduction of tumour lesion size in the subject.

31. The combination for use of any one of the preceding claims, wherein administration of the antibody-cytolysin conjugate and the ICI increases progression free survival and/or overall survival time of the subject, relative to a control subject who has been treated with the antibody-cytolysin conjugate or the ICI as monotherapies, but not in combination.

32. The combination for use of any one of the preceding claims, wherein the method comprises administration of at least 2mg/kg of the antibody-cytolysin conjugate to the subject, optionally at least 4mg/kg of the antibody-cytolysin conjugate to the subject, further optionally at least 7 mg/kg of the antibody-cytolysin conjugate to the subject.

33. The combination for use of any one of the preceding claims, wherein the method comprises administration of at least 100mg, at least 150mg, at least 200mg, at least 250mg, at least 300mg, at least 350mg or at least 400mg of the ICI to the subject.

34. The combination for use of any one of the preceding claims, wherein the method comprises administration of 200mg of ICI to the subject.

35. The combination for use of any one of the preceding claims, wherein the primary cancer and/or metastasis comprises a level of FAP expression detectable by immunohistochemistry.

36. A method of treating metastatic cancer in a mammalian subject, the method comprising administering (i) an anti-Fibroblast Activating Protein α (FAP) antibody-cytolysin conjugate having the formula A-(L-D)ₚ or a pharmaceutically acceptable salt or solvate thereof, wherein A is an anti-FAP antibody that selectively binds FAP, L is a linker, D is a drug comprising a cytolysin and p is 1 to 10, to the subject simultaneously, sequentially or separately with (ii) an immune checkpoint inhibitor (ICI), wherein the metastatic cancer comprises a primary cancer and metastasis.

37. The method of claim 36, wherein the primary cancer of the metastatic cancer comprises gastrointestinal cancer or lung cancer.
